# EUROPEAN PATENT APPLICATION

(11) **EP 2 119 396 A1**
(43) Date of publication of application: **18.11.2009**
(21) Application number: 08720839.3
(22) Date of filing: 25.02.2008
(51) Int. Cl.: A61B 5/151, A61B 5/1473, A61B 5/157

(54) **SENSOR DEVICE**

(30) Priority: 26.02.2007 JP 2007046323; 26.02.2007 JP 2007046327; 31.07.2007 JP 2007200248
(71) Applicant: National Institute Of Advanced Industrial Science, Tokyo 100-8921 (JP); Sumitomo Electric Industries, Ltd., Osaka-shi, Osaka 541-0041 (JP)
(72) Inventor: FUJIMURA, Tsuyoshi, Tsukuba-shi Ibaraki 305-8562 (JP); NAKAMURA, Hideaki, Tsukuba-shi Ibaraki 305-8562 (JP); ISHIKAWA, Tomoko, Tsukuba-shi Ibaraki 305-8562 (JP); GOTOH, Masao, Tsukuba-shi Ibaraki 305-8562 (JP); KARUBE, Isao, Tsukuba-shi Ibaraki 305-8562 (JP); KAIMORI, Shingo, Osaka-shi Osaka 554-0024 (JP); KITAMURA, Takahiko, Osaka-shi Osaka 554-0024 (JP); NAKAJIMA, Hiroto, Osaka-shi Osaka 554-0024 (JP); HAYAMI, Hiroshi, Osaka-shi Osaka 554-0024 (JP); HOSOYA, Toshifumi, Osaka-shi Osaka 554-0024 (JP)
(74) Representative: Setna, Rohan P.
(86) International application number: PCT/JP2008/053198
(87) International publication number: WO 2008/105373

(57) **Abstract**

In a lancet-needle-integrated type sensor element in which a lancet needle is fixed to a sensor chip, blood drawn out of a fingertip is sufficiently introduced into a sample space of the sensor chip.

A sensor device 1 has a needle-integrated type sensor element 20 which is detachably mounted therein and which has a sensor chip 100, a lancet needle 300 fixed to an end portion of the sensor chip 100, and a flow path forming element 170 provided compressibly and restorably at the end portion of the sensor chip 100, to which the lancet needle 300 is fixed. The sensor device 1 includes a puncture mechanism (a drive spring 31) configured to cause the sensor element 20 to puncture a target, and a pressing mechanism (a pressure spring 32) configured to urge the sensor element 20 to the extent that the sensor element 20 is prevented from being detached from the target by a restoring force of the flow path forming element 170 after the target is punctured.

## Description

### Technical Field

The present invention relates to a sensor device. More particularly, the invention relates to a sensor device suitable for mounting therein a lancet-needle-integrated sensor element into which a biosensor chip and a lancet needle are integrated.

### Related Art

A procedure for collecting blood using a blood collection lancet device to measure a blood-glucose level is performed by a patient by himself. Generally, a lancet device consists of a lancet needle, such as a puncture needle, and a lancet main element for driving the lancet needle. The puncture needle is quickly pushed out of the lancet main element so as to hit and puncture a fingertip or the like to cause bleeding therefrom.

Hitherto, the measurement of a blood-glucose level has been performed using such a lancet device and a measuring device separated therefrom. However, in recent years, there has been proposed a lancet-needle-integrated sensor device enabled to perform operations from blood collection to blood-glucose-level measurement in a single procedure by combining and integrating a biosensor with a lancet device.

For example, a sensor device described in Patent Document 1 consists of a chip main element having an internal space, and of a device main element in which a lancet element having a lance needle and the chip main element are mounted. The lancet element is manufactured to be able to move in the internal space of the chip main element. This sensor device is configured such that after the lancet element is housed in the chip main element, the chip element is set in the device main element, and that a tip end of the lancet needle is protruded from a sample introduction opening opened in an end portion of the chip main element.

The chip main element consists of a substrate having a concave portion which constitutes the internal space, and of a cover on which a biosensor is formed. A pair of electrodes, and terminal portions respectively connected to the pair of electrodes by wiring, which constitute the biosensor, are provided on the inner surface of the cover. The electrodes are provided to face a groove-like sample space extending into the internal space from the sample introduction opening. The biosensor is constituted by the electrodes and a reagent layer applied onto the surface of each of the electrodes.

However, the sensor device is configured so that the needle is moved in the sample space between the substrate and the cover, and that blood is introduced into the sample space from the sample introduction opening provided in the end portion of the tip main element. Thus, the thickness of the chip main element is increased. In addition, the sample introduction opening is relatively large. Thus, an associated large amount of blood is needed. This results in substantially high pain of a patient.

On the other hand, sensor chips have been developed, which are very small and can be mass-produced by a simple method. A biosensor chip described in Patent Document 2 has a structure similar to that of the biosensor chip illustrated in FIG. 4, and is configured such that a sample introduction opening is formed in an end portion of the chip by bending insulating plate members to form a cover and a substrate and by sticking both the cover and the substrate to each other with an adhesive layer so as to form a sample introduction opening in an end portion of the chip and so as to form a sample space between both the cover and the substrate, into which blood is introduced. Then, an electrode and another electrode connected by wiring to the former electrode are formed in the plate member by a method such as a sputtering method. A biosensor is constituted by the electrodes provided in the vicinity of the sample introduction opening, and the sample layer applied to the vicinity of the surface of each of the electrodes. This sensor chip has a structure in which the plate members are stuck to each other. Thus, the sensor chip is manufactured so as to be very thin and as to be compact. Consequently, an amount of necessary blood is reduced. Accordingly, it is expected that a physical burden to a patient can considerably be reduced.

Patent Document 1: International Publication No. WO 02/056769
Patent Document 2: International Publication No. WO 05/010519

### Disclosure of the Invention

### Problems to be solved by the Invention

Meanwhile, in a case where a lancet needle, such as a needle, is integrated with a biosensor chip, in order to introduce blood from the sample introduction space of the biosensor chip to the sample space, it is necessary that blood is sufficiently introduced into the sample introduction opening. However, in the case where the lancet needle is integrated with the biosensor chip using the thin biosensor as described in Patent Document 2, it is difficult to implement such a configuration that a needle having stiffness sufficient to puncture skin (i.e., the needle requires a certain thickness) is inserted into the sample introduction opening from the sample space, and that a tip end of the needle is once protruded from the sample introduction opening and subsequently housed in the sample space again, similarly to the sensor chip described in Patent Document 1.

Thus, it can be considered that the needle is fixed to the top surface of the cover of the biosensor chip described in Patent Document 2 or to the bottom surface of the substrate thereof. However, the sample introduction opening of this biosensor chip is very narrow. Thus, there are fears that blood may be insufficiently introduced into the sample introduction opening only by bringing the blood into contact with the sample introduction opening, and that because the sample introduction opening is distant from the tip end (or the axis) of the needle, blood coming out thereto may insufficiently be introduced to the sample introduction opening.

On the other hand, it is also considered that the needle is sandwichedly fixed between the bent plate members. In this case, the sensor device is configured such that the needle is protruded out of the sample introduction opening. Thus, it can be considered that blood can easily be guided, as compared with the case of fixing the needle to the top surface (or the bottom surface) of the biosensor chip. However, when the needle is taken out of a fingertip, the sensor chip is detached from the fingertip. Consequently, blood coming out therefrom may insufficiently be introduced to the sample introduction opening.

In each of the above cases, in order to locate the sample introduction opening of the sensor chip closer to blood (or a blood drop) coming from a punctured part, it is considered that a lancet needle taken out of skin is moved closer to the punctured part to thereby put blood in contact with the sample introduction opening. However, when the lancet needle is pushed by a strong force, the lancet needle is into the punctured part again. Thus, bleeding is stopped. Consequently, it is considered that in addition to the problem of insufficient blood supply to the sample space, another problem of giving pain to a user again (double puncture) occurs.
It is necessary for inserting the needle into the sample introduction opening to increase the thickness of the plate member. Thus, the size of the sensor chip has to be increased. Consequently, it is considered as another problem that an amount of blood to be collected cannot sufficiently be reduced.

The invention is accomplished in view of the above background art. An object of the invention is to assure, even when a lancet needle, such a puncture needle, is taken out of a fingertip, a flow path of blood between a puncture-needle mark and a sample introduction opening of a sensor chip in a sensor device in which a lancet-needle-integrated sensor element having the lancet needle fixed to the sensor chip is mounted, to impregnate the flow path with blood, and to half-forcibly introduce blood into a sample space, and to efficiently introduce blood in the sample space in reduced-pressure environment.

### Means for solving the Problems

According to an aspect of the invention, there is provided a sensor device for detachably mounting a needle-integrated type sensor element, the sensor element having a sensor chip, a lancet needle fixed to an end portion of the sensor chip, and a flow path forming element provided compressibly and restorably at the end portion of the sensor chip, to which the lancet needle is fixed,
the sensor device including:
a puncture mechanism for causing the sensor element to puncture a target; and
a pressing mechanism, which is different from the puncture mechanism, for urging the sensor element to an extent that the sensor element is prevented from being detached from the target by a restoring force of the flow path forming element after the target is punctured.

According to another aspect of the invention, there is provided a sensor device having a needle-integrated type sensor element detachably mounted therein, the sensor element having a biosensor chip, and a lancet needle fixed to one end portion of the biosensor chip,
the sensor device including:
a puncture mechanism for forming an airtight space by applying a puncture target surface thereto and for causing the sensor element to hit the puncture target surface in the airtight space; and
a decompressing mechanism for decompressing the enclosed airtight space, wherein
the airtight space is decompressed by driving the decompressing mechanism; and
a sample is guided into a sample space from a sample introduction opening of the biosensor chip.

### Advantages of the Invention

According to the invention, a user can sufficiently introduce blood into a sample introduction opening without performing double puncture. Accordingly, favorable measurement can be performed even when using a thin compact biosensor chip disclosed in Patent Document 2. That is, the measurement can be performed using a small amount of blood. In addition, a sensor device itself can be miniaturized.
Even when the needle hits a surface of a fingertip or the like, which is to be punctuated, the biosensor chip is placed in the reduced-pressure environment. Thus, element fluid serving as a sample flowing out of the surface to be punctuated becomes easily guided into the sample space. Accordingly, favorable measurement can be performed even when using a thin compact biosensor chip disclosed in Patent Document 2. That is, the measurement can be performed using a small amount of blood.

### Brief Description of the Drawings

[FIG. 1]

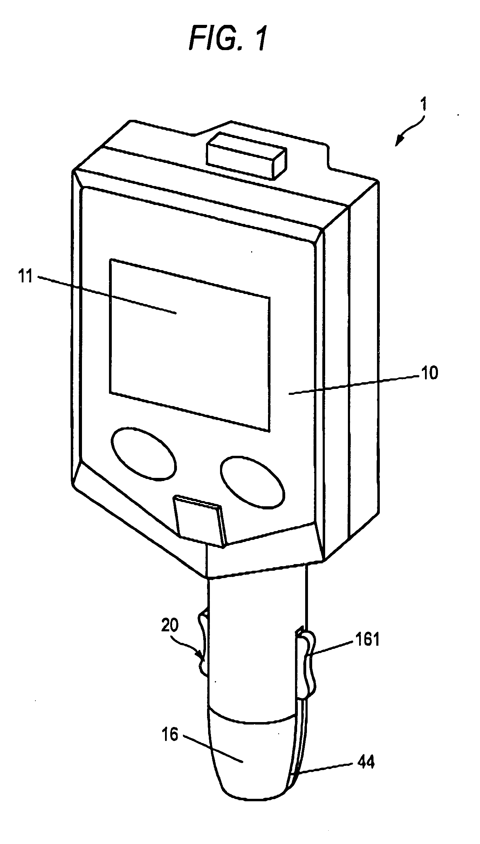

   FIG. 1 is a schematic perspective view of a sensor device according to a first embodiment of the invention.
[FIG. 2]

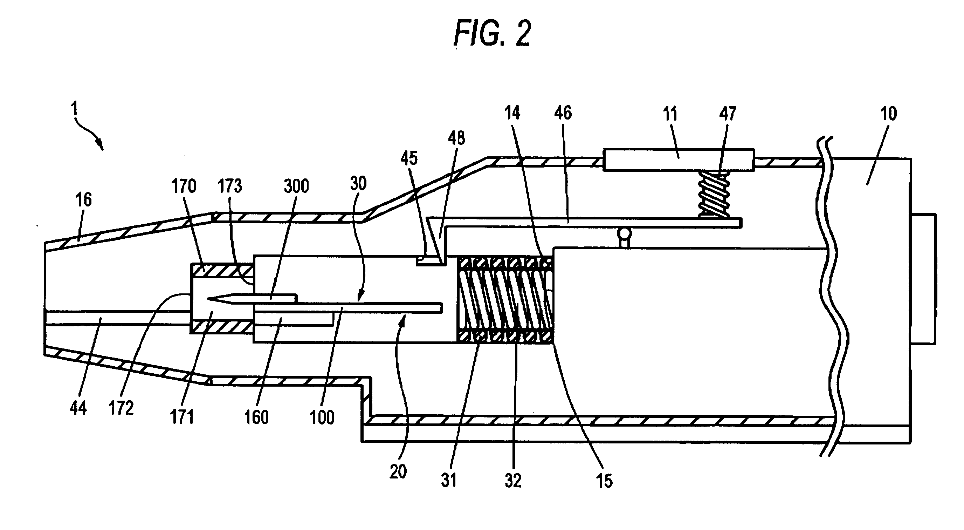

   FIG. 2 is a schematic view of an internal structure of the sensor device according to the first embodiment, in which a sensor element is mounted.
[FIG. 3]

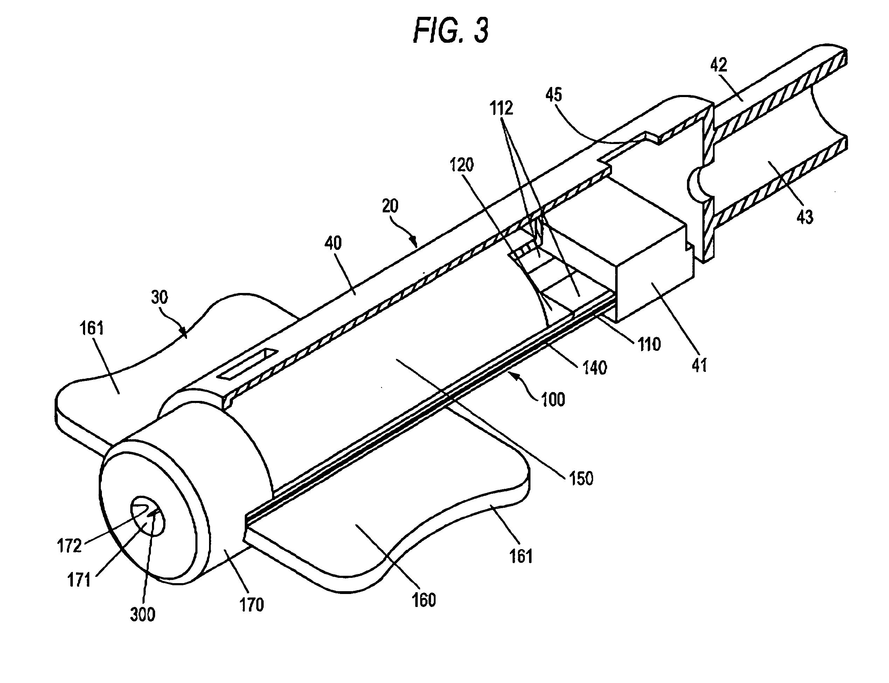

   FIG. 3 is a partially broken perspective view illustrating a sensor element to be mounted in the sensor device according to the first embodiment.
[FIG. 4]

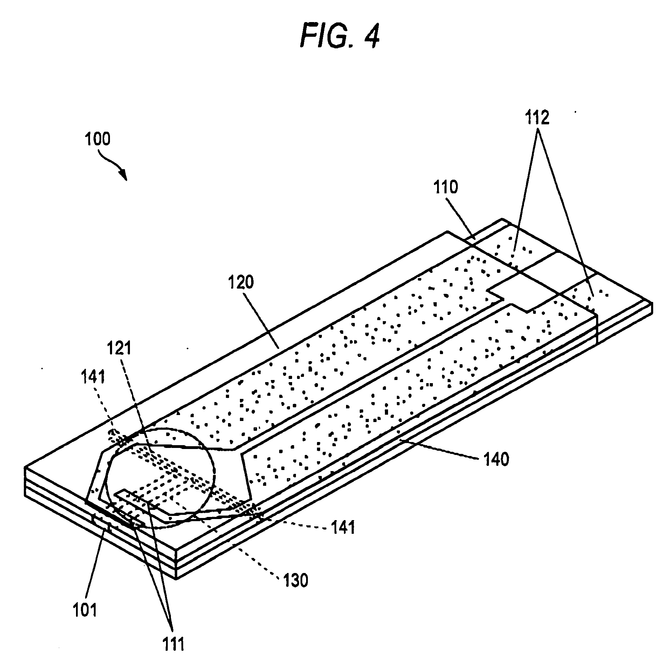

   FIG. 4 is a schematic perspective view of a sensor chip constituting a sensor main element to be mounted in the sensor device according to the first embodiment.
[FIG. 5]

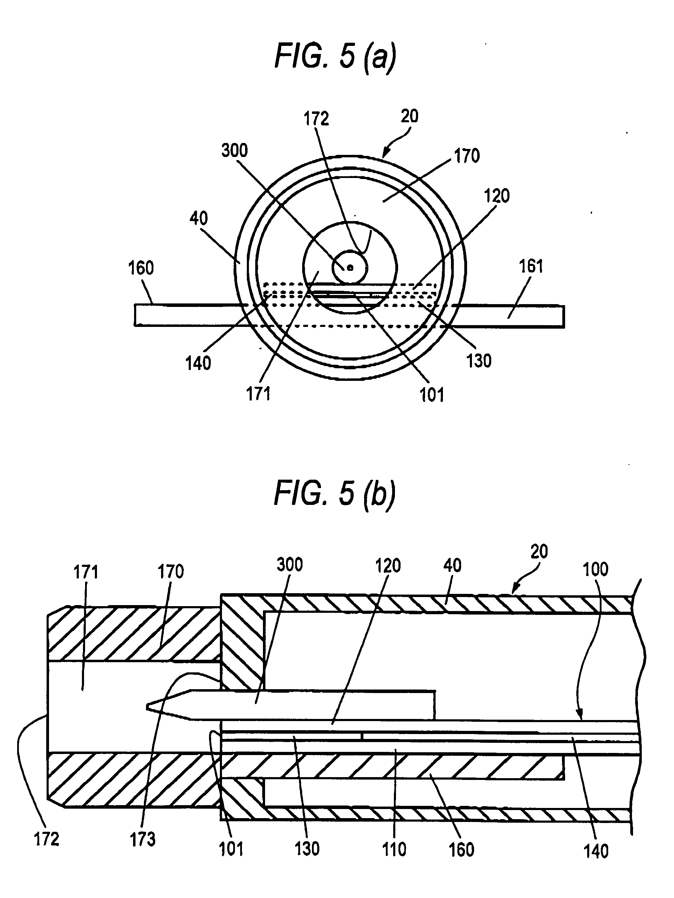

   FIG. 5 is a partially enlarged schematic explanatory view illustrating the sensor main element to be mounted in the sensor device according to the first embodiment. FIG. 5 (a) is a plan view thereof. FIG. 5(b) is a cross-sectional view thereof.
[FIG. 6]

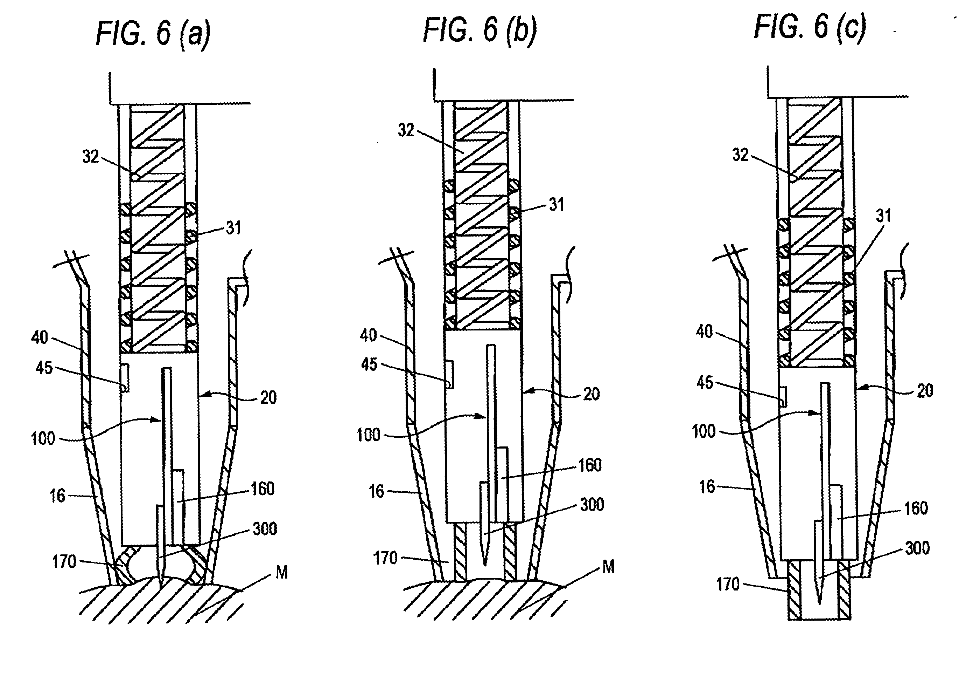

   FIGS. 6 (a) to 6 (c) are explanatory view illustrating an operation of the sensor device according to the first embodiment of the invention.
[FIG. 7]

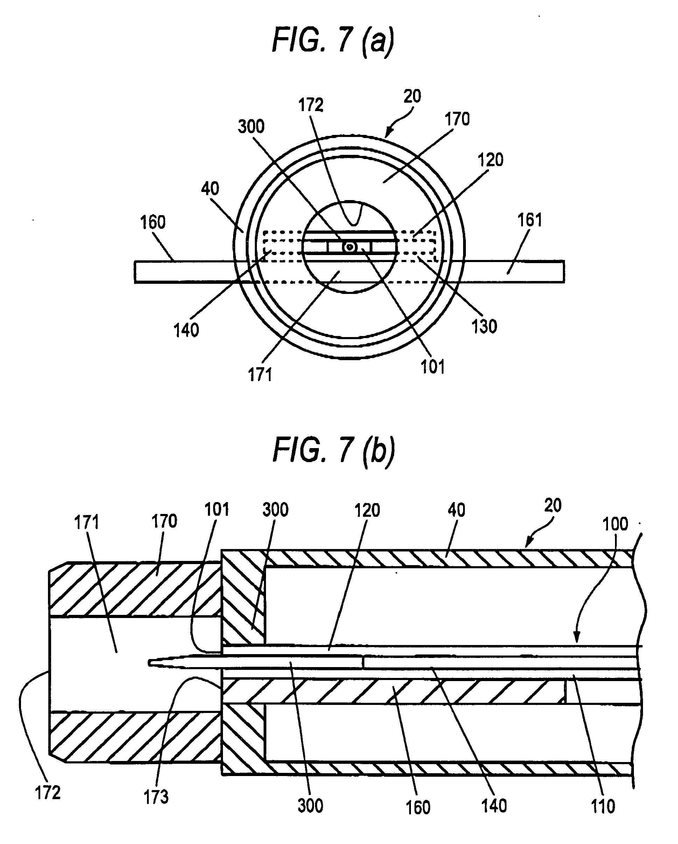

   FIG. 7 is a partially enlarged schematic explanatory view of another example of the sensor main element to be mounted n the sensor device according to the first embodiment. FIG. 7(a) is a plan view thereof. FIG. 7(b) is a cross-sectional view thereof.
[FIG. 8]

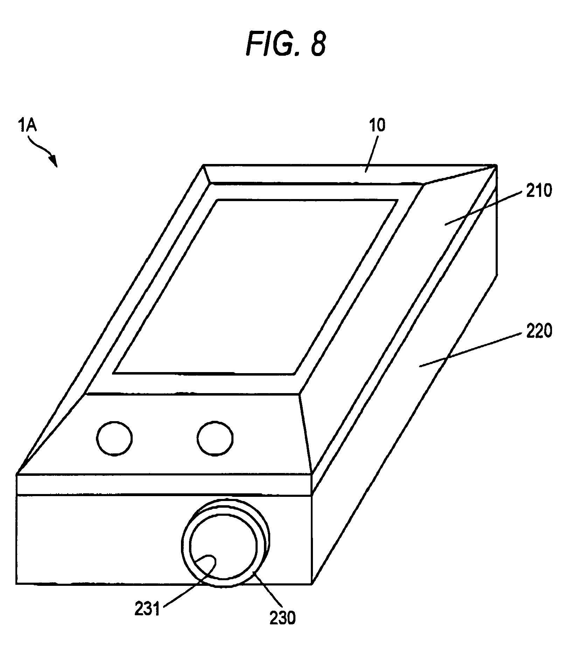

   FIG. 8 is a schematic perspective view of a sensor device according to a second embodiment of the invention.
[FIG. 9]

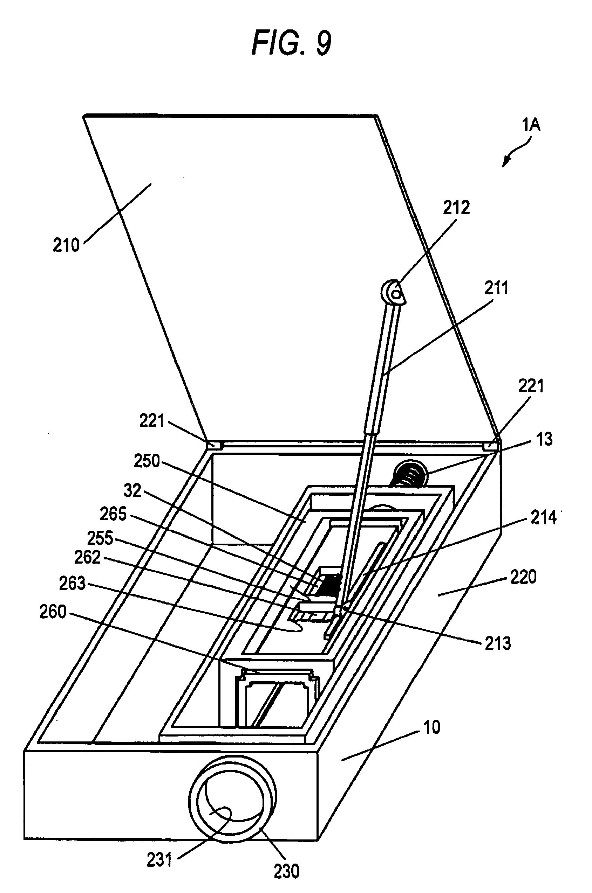

   FIG. 9 is a schematic perspective view illustrating.a state in which a lid illustrated in FIG. 8 is opened.
[FIG. 10]

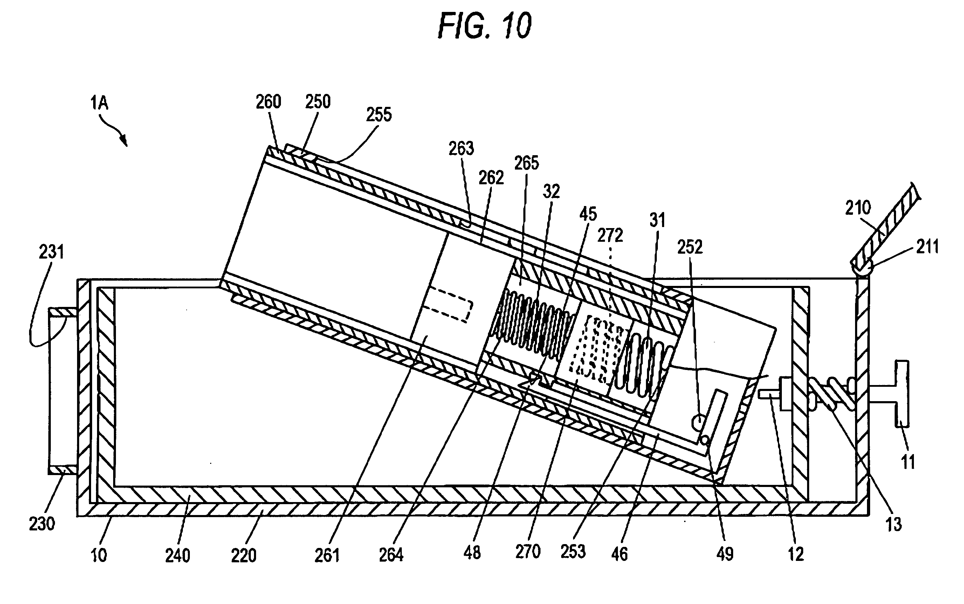

   FIG. 10 is a schematic cross-sectional view illustrating a state in which the lid illustrated in FIG. 8 is opened.
[FIG. 11]

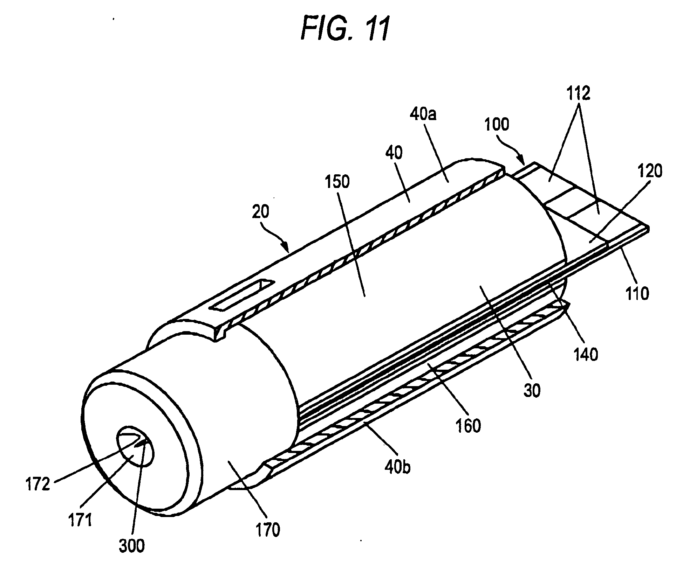

   FIG. 11 is a partially broken schematic perspective view of a sensor element to be mounted in the sensor device illustrated in FIG. 8.
[FIG. 12]

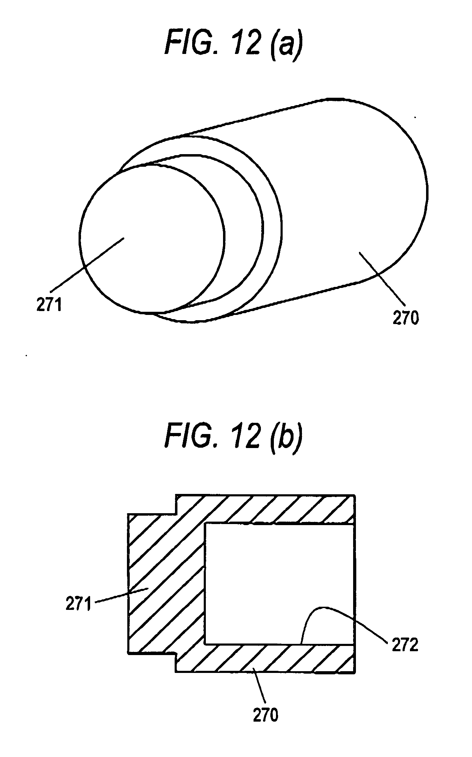

   FIG. 12 is a view illustrating a spring connector which connects a drive spring of the sensor device illustrated in FIG. 8 to a pressure spring thereof. FIG. 12(a) is a perspective view thereof. FIG. 12(b) is a cross-sectional view thereof.
[FIG. 13]

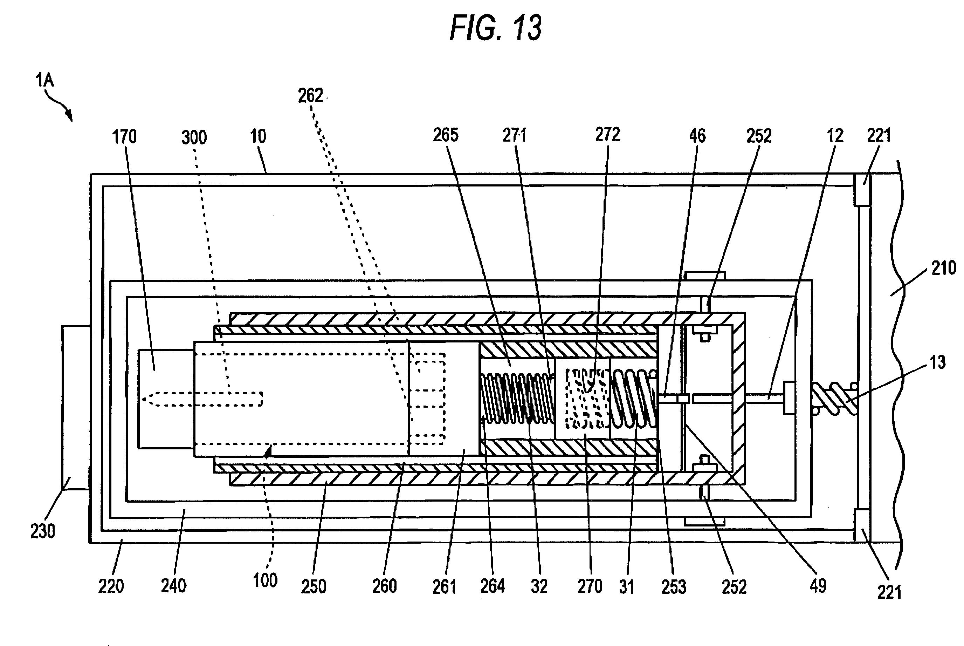

   FIG. 13 is a schematic plan view of the sensor device illustrated in FIG. 8, in which a sensor element is mounted and a lid is opened.
[FIG. 14]

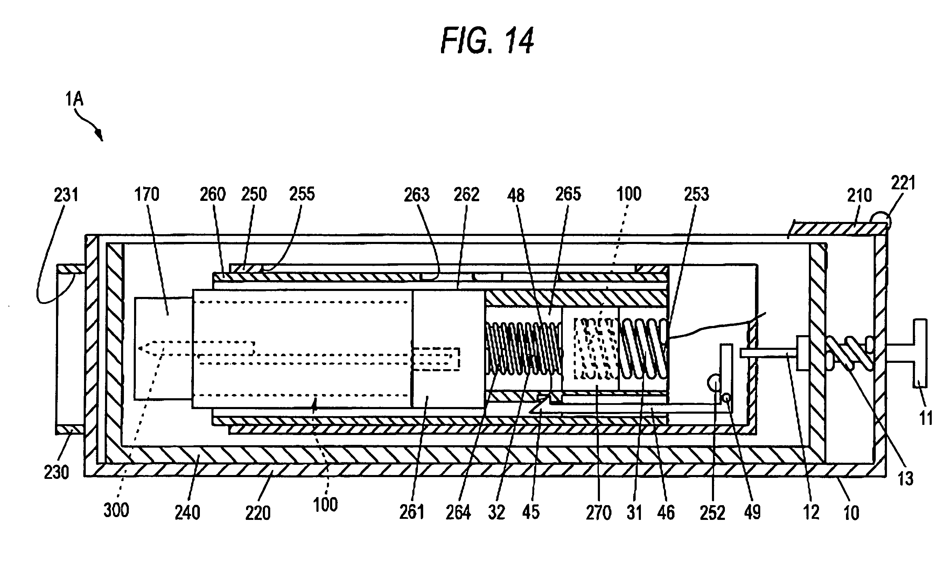

   FIG. 14 is a schematic cross-sectional view of the sensor device illustrated in FIG. 8, in which the sensor element is mounted and the lid is opened.
[FIG. 15]

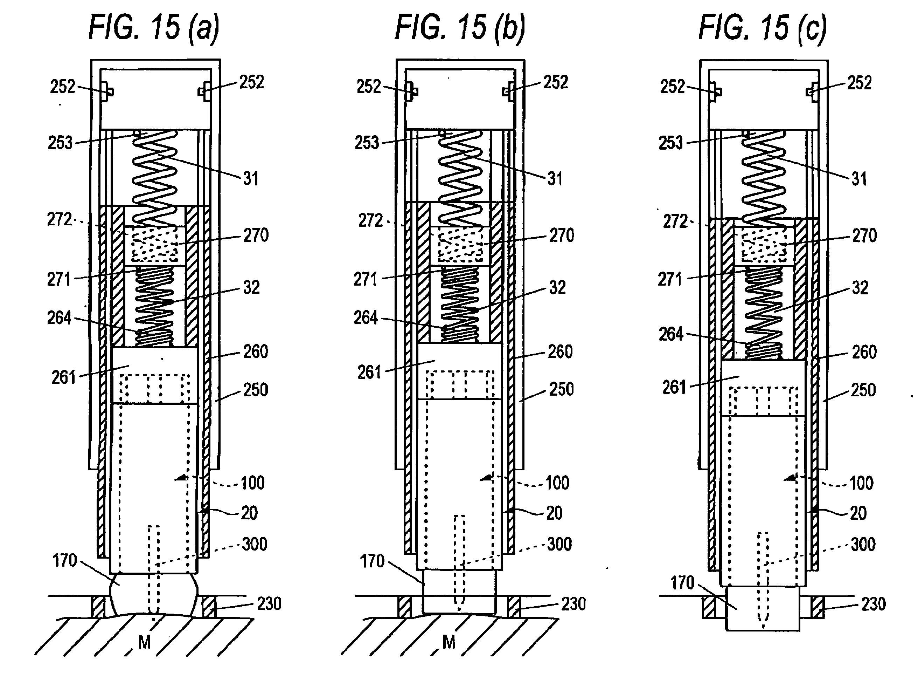

   FIGS. 15(a) to 15(c) are explanatory view illustrating an operation of the sensor device illustrated in FIG. 8.
[FIG. 16]

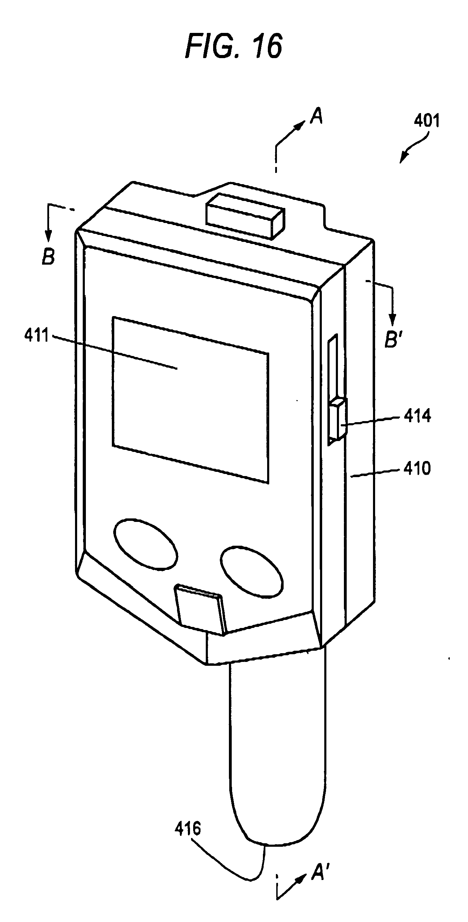

   FIG. 16 is a schematic perspective view illustrating a sensor device according to a third embodiment of the invention.
[FIG. 17]

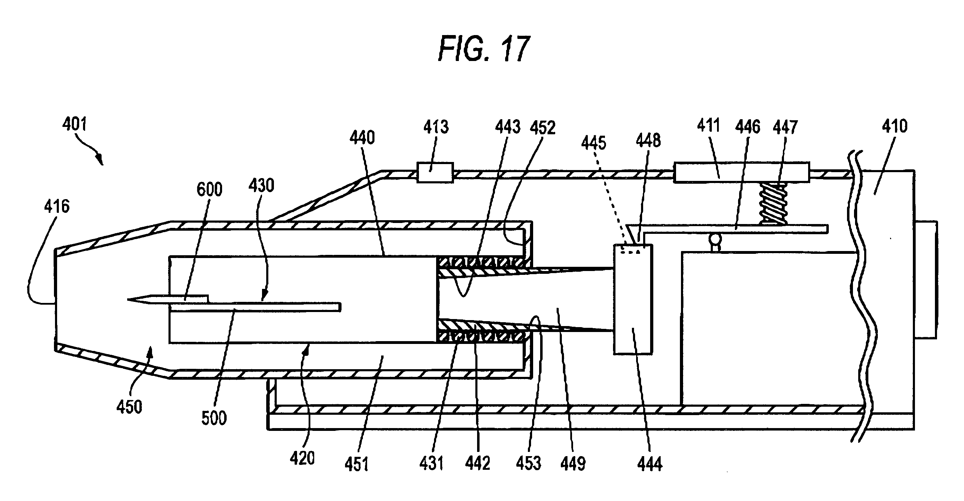

   FIG. 17 is a schematic cross-sectional view, taken on line A-A' shown in FIG. 16, illustrating a structure of the sensor device according to the third embodiment in which a sensor element is mounted.
[FIG. 18]

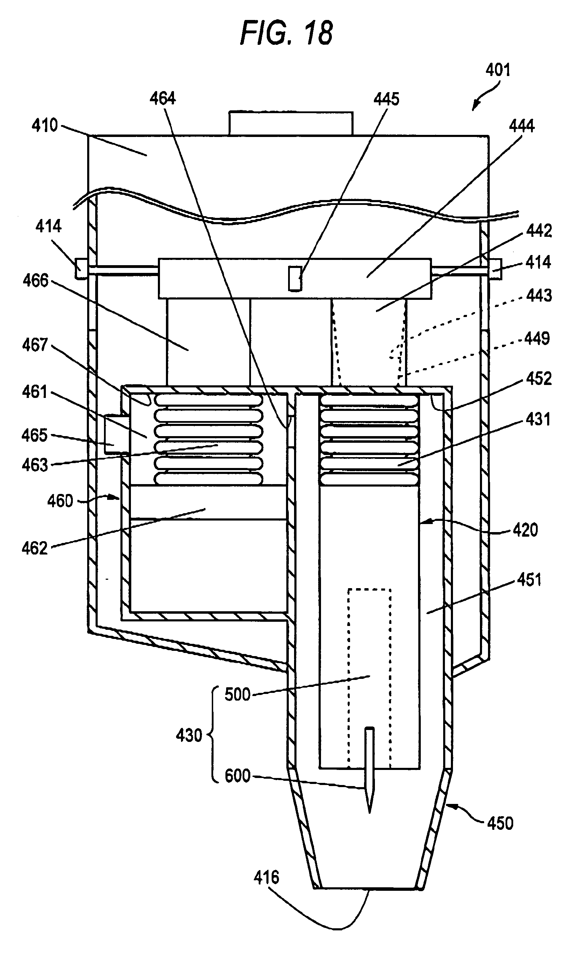

   FIG. 18 is a schematic cross-sectional view, taken on line B-B' shown in FIG. 16, illustrating the structure of the sensor device according to the third embodiment in which the sensor element is mounted.
[FIG. 19]

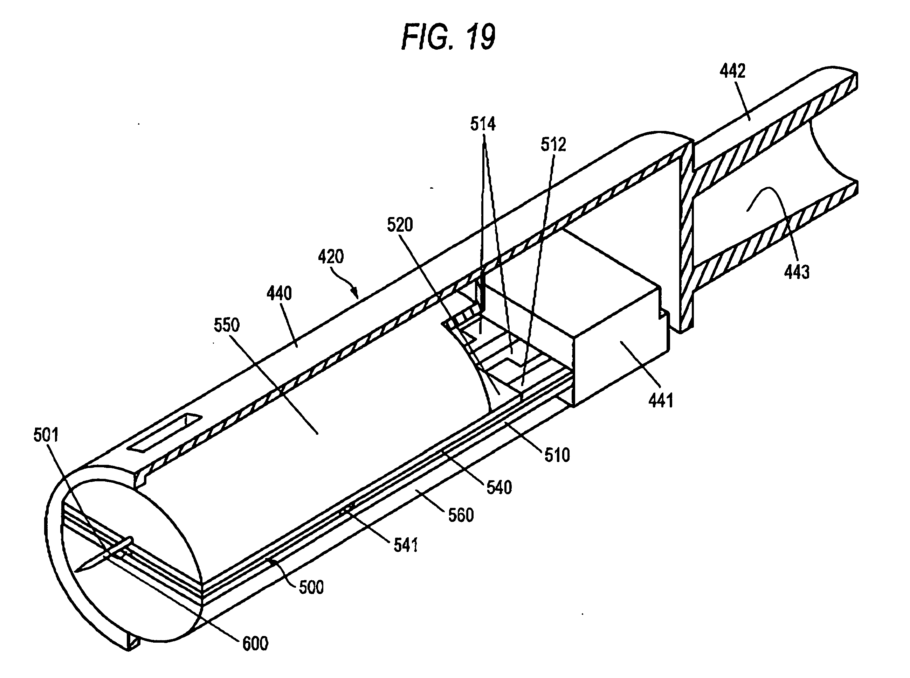

   FIG. 19 is a partially broken schematic perspective view illustrating the sensor element mounted in the sensor device according to the third embodiment.
[FIG. 20]

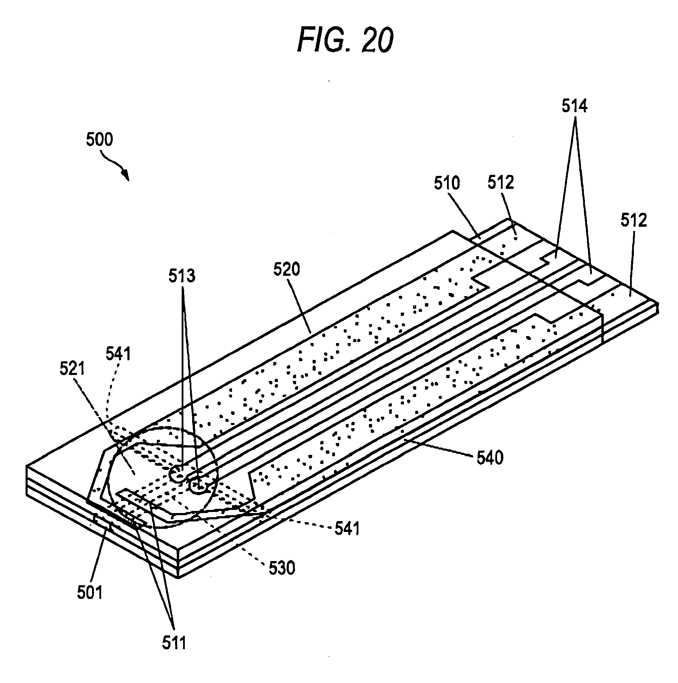

   FIG. 20 is a schematic perspective view illustrating a sensor chip constituting the sensor element mounted in the sensor device according to the third embodiment.
[FIG. 21]

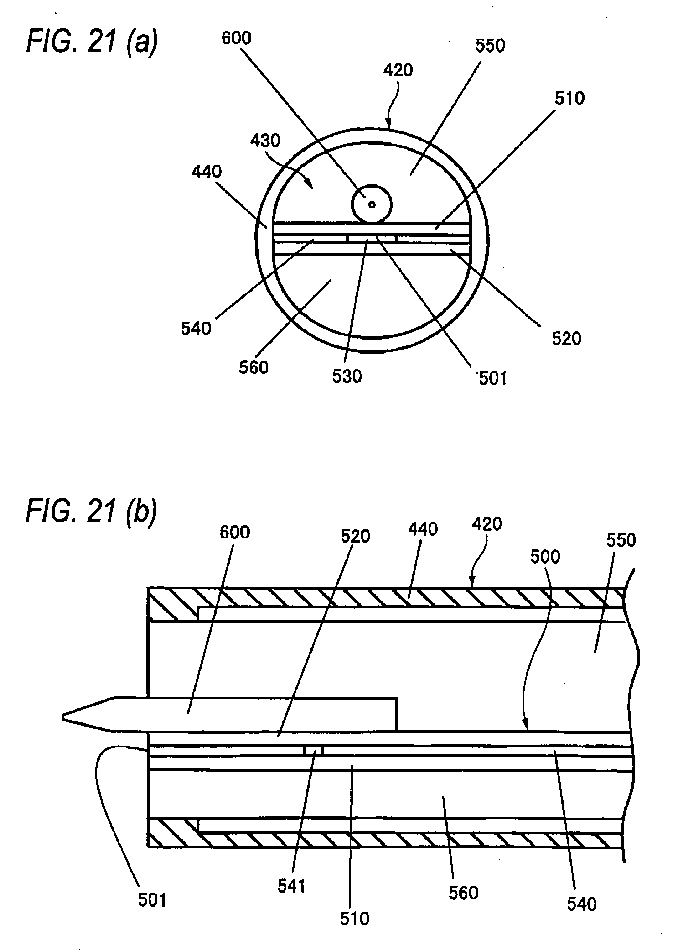

   FIG. 21 is a partially enlarged schematic explanatory view of the sensor element mounted in the sensor device according to the third embodiment. FIG. 21(a) is a plan explanatory view thereof. FIG. 21(b) is a cross-sectional explanatory view thereof.
[FIG. 22]

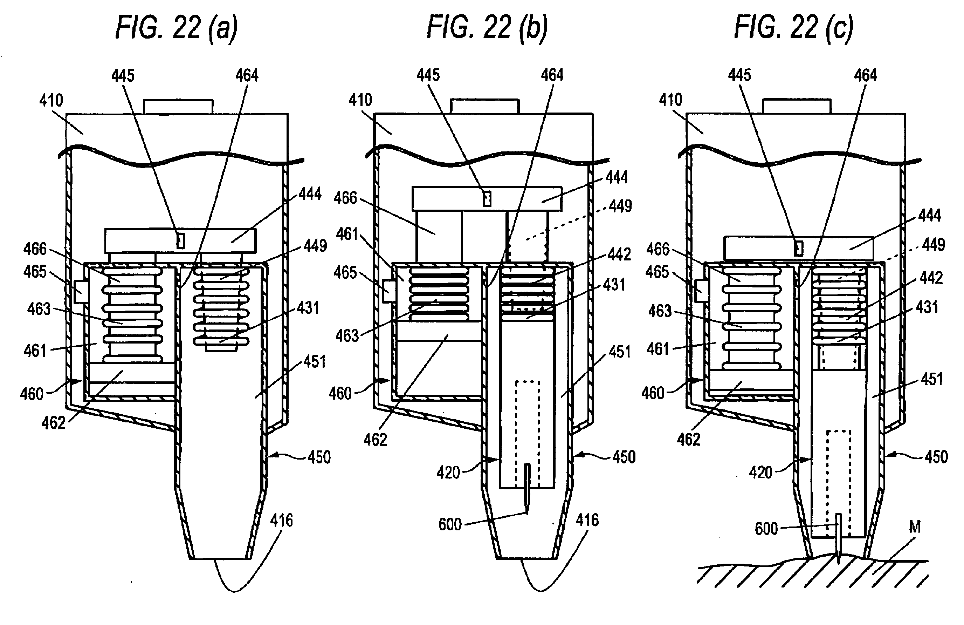

   FIGS. 22(a) to 22(c) are explanatory view illustrating an operation of the sensor device according to the third embodiment.
[FIG. 23]

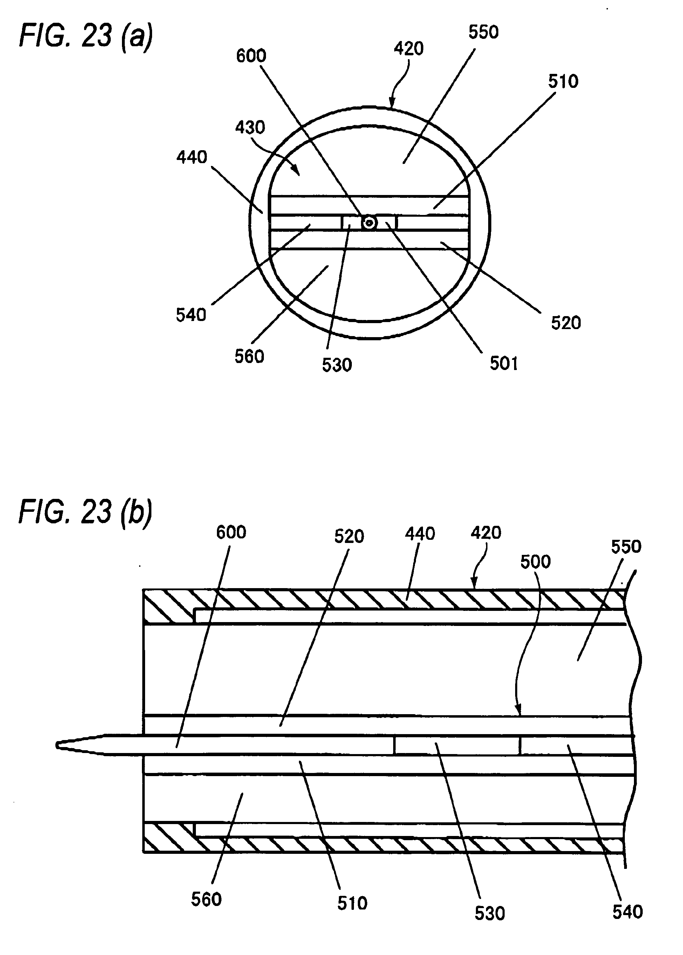

   FIG. 23 is a partially enlarged schematic explanatory view of another example of a sensor main element of the sensor device according to the third embodiment. FIG. 23 (a) is a plan explanatory view thereof. FIG. 23(b) is a cross-sectional explanatory view thereof.

### Description of Reference Numerals and Signs

1, 401 sensor devices
10, 410 device elements
11, 411 drive buttons
15 wall surface of a device element that fixes an end of a pressure spring
16 cap element
20, 420 sensor elements
30 sensor main element
31, 430 drive springs
32 pressure spring
40, 440 sensor bodies
41, 441 connectors
42, 442 drive spring mounting portions
43 pressure spring mounting portion
44 guide slit
45 concave portion
46, 446 hook members
47 holding spring
48, 448 hooks
100, 500 sensor chips
101, 501 sample introduction openings
110, 510 substrates
120, 520 covers
121, 521 reagent layers
130, 530 sample spaces
140, 540 adhesive agent layers
150 fixing member
160 support member
161 wing portion
170 flow path forming element
171 flow path
172 puncture hole
173 rear surface opening portion
210 lid element
220 casing element
240 fixed frame
250 fixed cassette
260 movable cassette
270 spring connector
300, 600 lancet needles
414 lock lever
416 opening in an end part of the device element
443 sensor element holding portion
444 continuous connection portion
445 concave portion
449 holding member
450 puncture mechanism portion
451 puncture mechanism chamber
460 reduced-pressure mechanism portion
461 cylinder chamber
462 piston
463 piston spring
464 communication hole
465 electromagnetic valve
466 piston rod
541 air hole

### Best Mode for Carrying Out the Invention

Hereinafter, the invention is more specifically described with reference to the accompanying drawings. FIG. 1 is a schematic perspective view of a sensor device 1 according to a first embodiment of the invention. FIG. 2 is a schematic view of an internal structure of the sensor device 1 according to the first embodiment, in which a lancet-needle-integrated sensor element 20 is mounted. FIG. 3 is a partially broken perspective view illustrating the sensor element 20. FIG. 4 is a schematic perspective view of a sensor chip 100 constituting a sensor main element 30. FIG. 5 is a partially enlarged schematic explanatory view illustrating the sensor main element 30. FIG. 6 is an explanatory view illustrating an operation of the sensor device 1.

The sensor device 1 according to the first embodiment of the invention includes a device main element 10 in which the sensor element 20 having the sensor chip 100 and a lancet needle 300 is detachably mounted. The sensor element 20 includes the sensor main element 30 formed integrally with the sensor chip 100 and the lancet needle 300, and a sensor body 40 to be attached to the device main element 10. The sensor main element 30 is constructed by integrating the lancet needle 300, which includes a puncture needle for hurting skin to cause bleeding or a miniature cutting tool, and the sensor chip 100 with each other by a fixing member 150 and a support member 160. Further, the sensor element 20 is provided as what is called a disposable product.

As illustrated in FIG. 4, the sensor chip 100 has a structure in which a cover 120 and a substrate 110, each of which is formed of an insulating plate member, are stuck to each other with an adhesive agent layer 140 so as to form a sample space 130 between the cover 120 and the substrate 110. A sample introduction opening 101 connected to the sample space 130 is opened in an end part of the sensor chip 100. An air hole 141 connected to the sample space 130 and the outside of the sensor chip 100 is provided at a rear end part of the sample space 130 so as to extend to both sides of the rear end part of the sample space 130. A pair of electrodes 111 is formed on the substrate 110 so as to face the sample space 130. Voltage taking-out terminals 112 electrically connected to the electrodes 111 with electrical conductors, respectively, are formed at an end part of the substrate 110, which is opposite to the sample introduction opening 101. The cover 120 is provided with a reagent layer 121 which reacts with a sample (more specifically, body fluid such as blood) and is formed to face the sample space 130. A biosensor includes the pair of electrodes 111 and the reagent layer 121. A chip including the substrate and the cover manufactured by bending plate members as described by the Patent Document 2 is exemplified as the sensor chip 100. However, according to the invention, a structure other than that described by the Patent Document 2 can be employed as the structure of the biosensor. As long as a structure is such that the sample introduction opening 101 is provided between the substrate 110 and the cover 120 and that a sample is introduced from the sample introduction opening 101, the invention can be applied to such a structure.

The lancet needle 300 is arranged on the cover 120 of the sensor chip 100 and is sandwiched between a fixing member 150 and a support member 160 and fixed to the sensor chip 100. The lancet needle 300 is fixed thereto by protruding a tip end of the lancet needle 300 from a tip end of the sensor chip 100 in order to hurt skin (target). The lancet needle 300 is arranged such that an axis of the lancet needle 300 is substantially parallel to a direction in which the sample space 130 is provided so as to quickly guide to the sample introduction opening 101 body fluid that comes out from the skin, and that the axis thereof is located close to, preferably, just above the sample introduction opening 101. According to the invention, as will be described below, a flow path forming element 170 is attached thereto. Thus, as long as the lancet needle is located in (a transversal cross-section of) a flow path 171 formed by attaching the flow path forming element, the lancet needle can be located somewhat distant from the sample introduction opening 101.

The support member 160 has a function of supporting the sensor chip 100 from the bottom surface thereof, and is made of, e.g., a plastic material. The support member 160 has a wing portion 161 configured to be easily fixed to a sensor body 40 and attached to and detached from the device main element 10 and to be easily pinched by two fingers. The fixing member 150 has a function of protecting the sensor chip 100 from the direction of the top surface thereof, and is manufactured by being made of, e.g., a plastic material. Also, the fixing member 150 has a function to fix the lancet needle 300. A cutout (not shown) for fixing the lancet needle 300 is formed in the bottom surface. Apparently, the fixing member 150 and the support member 160 can be formed integrally with each other.

The sensor main element 30 is attached to the sensor body 40 formed like a substantially cylinder, as illustrated in, e.g., FIG. 3. The sensor body 40 is used to attach the sensor chip to the device main element 10. In the sensor body 40, a connector 41 for electrically connecting the terminals 112 of the sensor chip 100 to a measuring circuit (not shown) of the device main element 10 is provided. A cylindrical drive spring mounting portion 42, whose diameter is smaller than that of a leading end side part of the sensor body 40 is provided at a rear end side of the sensor body 40. An inner part of the cylinder is formed as a pressure spring mounting portion 43.

As illustrated in FIGS. 2 and 3, the flow path forming element 170 is provided on the front surface of the sensor element 20. As will be described below, the flow path forming element 170 serves to quickly guide body fluid flown out of a punctured part to the sample introduction opening 101. The flow path forming element 170 illustrated in the drawings is manufactured substantially like a cylinder. The inside of the cylinder is a flow path 171 in which the body fluid flows. A leading end opening of the flow path forming element 170 is formed as a puncture hole 172 through which a tip end of the lancet needle 300 is inserted. The flow path forming element 170 is configured such that the axis of the lancet needle 300 and the sample introduction opening 101 are located in a rear surface opening portion 173. The rear surface opening end of the flow path forming element 170 is attached closely to the front surface of the sensor element 20 (the sensor main element 30) so that the body fluid does not leak from the flow path 171 to outside. A method of attaching the flow path forming element 170 is considered as, e.g., a method of providing a projection (not shown) on each of the fixing member 150 and the support member 160 and then latching the sensor element 20 and the flow path forming element 170 together by capping the leading end of the sensor element 20 with the rear surface opening portion 173 of the flow path forming element 170, i.e., latching the projections 151 and the flow path forming element 170, alternatively, capping the leading end of the sensor body 40 with the rear surface opening portion 173 of the flow path forming element 170. However, the flow path forming element 170 can be formed such that the flow path 171 is formed therein, or that the leading end opening is covered with a cap part in which the puncture hole 172 is opened.

The flow path forming element 170 is manufactured to have a size sufficient to the extent that the tip end of the lancet needle 300 is located inwardly from the puncture hole 172 when the flow path forming element 170 is attached to the sensor main element 30 in a natural state. The flow path forming element 170 has a function of a protection cap for the lancet needle 300. The flow path forming element 170 is made of a polymer material having airtightness and elasticity, e.g., rubber such as silicon rubber, urethane rubber, or acrylic rubber, or rubber or sponge contacting a single polymer of ethylene, styrene, or the like, or copolymers thereof, polyolefin such as polyethylene and polypropylene, polyester such as polyethylene terephthalate (PET) or polybutylene terephthalate (PBT), or fluorine resin such as polytetrafluoroethylene (PTFE) or perfluoroalkoxy polymer resin (PFA). When the body fluid does not permeate the polymer material, the degree of the airtightness of the polymer material is sufficient. It is necessary that an elastic force of the polymer material is sufficient to compress, when the sensor element 20 hits skin, the sensor element 20 so that the tip end of the lancet needle 300 is protruded from the puncture hole 173, and to allow the sensor element 20 to restore to the extent that the lancet needle 300 inserted into the skin comes out therefrom. Body fluid flowing out of a punctured part (corresponding to a puncture mark) where the lancet needle 300 is removed is guided from the flow path 171 formed in the flow path forming element 170 to the sample introduction opening 101.

The sensor element 20 having the flow path forming element 170 is attached to the device main element 10 by inserting the neighborhood part of the base end of each wing portion 161 through a guide slit 44 of the sensor body 40 (see FIG. 1). The device main element 10 includes a puncture mechanism for causing the sensor element 20 attached thereto to puncture skin, and a conversion circuit for converting an electromotive force generated in the sensor chip 100 to a measured value, and a display mechanism (not shown) for displaying the measured value, and the like. The puncture mechanism includes, e.g., a spring mechanism. The spring mechanism includes a drive spring 31 provided at a rear portion of the sensor element 20, and a holding mechanism for holding the drive spring 31 in a compressed state and for canceling, in response to a drive button 11, a held state in which the drive spring is held. The drive spring 31 is mounted in the sensor body 40 such that the drive spring mounting portion 42 is inserted into a leading-end-side part thereof. The drive spring 31 is housed in the device main element 10 to be able to push the other end thereof against a wall surface 14 located at a predetermined position in the device main element 10. The drive spring 31 illustrated in the drawing is not fixed to the device main element 10. When the compressed drive spring 31 is restored to a natural length, the drive spring 31 can be displaced by maintaining a state in which the length of the drive spring 31 is a natural length. Apparently, the drive spring 31 can be configured so that one end thereof is fixed to the device main element 10 (e.g., the wall surface 14).

The holding mechanism includes a concave portion 45 formed on the sensor body 40, a hook member 46 interlocked with the drive button 11, and a holding spring 47. The holding spring 47 urges the drive button 11 upwardly (in a pushing-up direction), so that a hook 48 provided at an end of the hook member 46 is caught in the concave portion 45. When the sensor element 20 is inserted into the device main element 10, the hook 48 provided at the end of the hook member 46 engages with the concave portion 45, so that the drive spring 31 is maintained in a compressed state. Next, when the drive button 11 is pressed, the hook 48 is disengaged from the concave portion 45. Thus; the drive spring 31 is released. Consequently, the sensor element 20 is frontwardly and swiftly pushed out by the restoring force of the drive spring 31. Consequently, the lancet needle 300 hitting the skin punctures overcomes the force of repulsion of the flow path flowing element 170, so that the leading end of the lancet needle 300 is inserted into the skin. Subsequently, the lancet needle 300 is removed from the skin by the restoring force of the flow path forming element 170. Body fluid coming out from the skin flows into the flow path 171.

The pressing mechanism is provided in the sensor device 1 according to the first embodiment of the invention in order to fill the flow path 171 of the flow path forming element 170 with body fluid, and to sufficiently assure the flow of body fluid. As long as the flowing path forming element is pressed against the skin, the flow path 171 can be assured by a mechanism until measurement is finished, this mechanism can be used as the pressing mechanism. For example, the pressure spring 32 is used as the pressing mechanism. The pressure spring 32 is mounted by inserting the leading end thereof into the pressure spring mounting portion 43 of the sensor element 20. The rear end of the pressure spring 32 is fixed to the predetermined wall surface 15 in the device main element 10. When the sensor element 20 is mounted in the device main element 10, the pressure spring 32 is housed in the device main element 10 in a compressed state and urges the sensor element 20 towards the tip end side. When the drive spring 31 is released by operating the drive button 11, the sensor element 20 (the flow path forming element 170) is urged in the direction of the skin by the resorting force thereof. Thus, the front surface of the flow path forming element 170 is caused to abut against the skin. That is, when the sensor element is operated only by the drive spring 31 without providing the pressure spring 32 therein, the lancet needle 300 is disengaged from the skin by the restoring force of the flow path forming element 170. Thus the body fluid flows into the flow path 171. However, the drive spring 31 is brought into a free condition. Consequently, the front surface of the flow path forming element 170 is detached from the skin, so that the flow of the body fluid (flow path) ends. Thus, there is a fear that the flow path 171 is insufficiently filled with the body fluid, and that a necessary amount of body fluid does not flow into the sample space 130. Consequently, according to the invention, the device is constructed so that the flow path forming element 170 is pressed against the skin by the pressure spring 32 even after the skin is punctured. Thus even after the lancet needle 300 is detached from the skin, the flow path 171 is sufficiently filled with blood. In order to enable such a function, it is desired that when the flow path forming element 170 is located in the vicinity of the skin just after the skin is punctured, at least the magnitude of the restoring force of the pressure spring 32 is substantially equal to or less than that of the restoring force of the flow path forming element 170. However, even when the magnitude of the restoring force of the pressure spring 32 is less than that of the flow path flowing element 170, at least the natural length of the pressure spring 32 is required to have a minimum value necessary to press the flow path flowing element 170 against the skin. As long as the flow path 171 is filled with body fluid and the flow of body fluid can be assured, the front surface of the flow path forming element 170 can be slightly detached from the skin. On the other hand, even when the magnitude of the restoring force of the pressure spring 32 is larger than that of the restoring force of the flow path forming element 170, the lancet needle can be removed from the skin due to the force of repulsion generated when the needle hits the skin. However, even in such a case, it is necessary that the restoring force of the pressure spring 32 is not so large that the lancet needle can be prevented from being inserted into the skin again.

In order to protect the sensor element 20 mounted in the device main element 10, usually, a cap element 16 is detachably attached to the end portion of the device main element 10 so that the cap element 16 is detachably provided at the leading end part of the device main element 10. When the skin is punctured, the puncturing is performed by pressing the leading end of the cap element 16 to a fingertip or the like. Thus, a puncture place is located inwardly from the leading end of the cap element 16. Accordingly, in a case where no cap element 16 is provided or where the entire device 1 is assumed to be disposable, the restoring force of the pressure spring 32 can be set sufficient to the above extent.

However, in a case where the device main element 10 is provided with the cap element 16, when the flow path forming element 170 is located in the cap element 16 after the skin is punctured, occurrence of unfavorable situations for repeatedly using the device main element 10 is assumed, in which body fluid adhering to the flow path forming element 170 may adhere to the cap element 16 and in which the body fluid may contaminate the inner surface of the cap element 16 when the sensor element 20 is removed.

Thus, preferably, when a fingertip is detached from the cap element 16, the device employs the pressure spring 32 having a restoring force sufficient to the extent that the front surface of the flow path forming element 170 can be located frontwardly from the leading end opening (projection opening) of the cap element 16, i.e., the pressure spring 32 having a natural length that is longer than the distance between the wall surface 15 to which the pressure spring 32 provided in the device main element 10 to the leading end of the cap element 16. Apparently, the device can employ the pressure spring 32 of a length at which the entire flow path forming element 170 is protruded from the leading end of the cap element 16. Consequently, when the fingertip is detached from the device 1 after the skin is punctured, the front surface of the flow path forming element 170 is located outwardly from the cap element 16. Consequently, the cap element 16, more particularly, the inner surface thereof can be prevented from being contaminated.

That is, in order to achieve these objects (i.e., to assure the flow path after the skin is punctured and to prevent the cap element 16 from being contaminated), regardless of the presence/absence of the cap element 16, the spring constant and the length of the pressure spring 32 and the elastic force of the flow path forming element 170 are adjusted comprehensively according to the structure of the device main element 10, e.g., the presence/absence of the cap element 16, the distance from the position, at which the pressure spring 32 is fixed, and the front surface of the flow path forming element 170, the length of the flow path forming element 170, and the like.

Next, an operation of the sensor device 1 according to the first embodiment of the invention is described hereinafter with reference to an explanatory view illustrating an operation thereof shown in FIG. 6. First, when the sensor element 20 to which the flow path forming element 170 is attached is deeply inserted into the device main element 10, the hook 48 of the drive mechanism is engaged with the concave portion 45 of the sensor body 40, so that the sensor element 20 is set at a predetermined position in a state in which the drive spring 31 and the pressure spring 32 are compressed (see FIG. 2). When the sensor element 20 is set in the device main element 10, the measuring circuit is turned on. When the drive button 11 is pressed in a state in which a fingertip is pushed against the leading end of the cap element 16, the hook 48 is disengaged from the concave portion 45, so that the drive spring 31 and the pressure spring 32 are stretched. Then, the sensor element 10 swiftly hits the fingertip. Thus, the tip end of the lancet needle 300 protruded from the puncture hole 172 of the compressed flow path forming element 170 is inserted into the fingertip (see FIG. 6(a)). Subsequently, the tip end of the lancet-needle 300 is removed from the fingertip by the restoring force of the flow path forming element 170. In addition, the front surface of the flow path forming element 170 is maintained by the restoring force of the pressure spring 32 in a state in which this front surface is substantially in contact with the fingertip (see FIG. 6(b)). Then, upon completion of measurement, when the fingertip is removed from the sensor device 1, the flow path forming element 170 is pushed out more frontwardly by the restoring force of the pressure spring 32, so that the flow path flowing element 170 is stopped in a state in which the front surface of the flow path forming element 170 is protruded from the leading end of the cap element 16 (see FIG. 6(c)). Subsequently, the sensor element 20 is removed from the device main element 10. Thus, the measurement is finished. When the sensor element 20 is removed from the device main element 10, the measuring circuit is turned off. The removed sensor element 20 is discarded.

Thus, in the sensor device 1 according to the first embodiment of the invention, the substantially cylindrical flow path forming element 170 compressible and restorable in a direction, in which the lancet needle 300 is inserted, is provided at the end of the sensor chip 100. The sensor device 1 is provided with the pressing mechanism, e.g., the pressure spring 32, which urges the leading end surface of the flow path forming element 170 in the direction of a fingertip to the extent that the tip end of the lancet needle 300 is not in contact with the fingertip after the fingertip is punctured. Thus, the flow path is surely assured, in which blood coming out between the punctured part and the sample introduction opening 101 of the sensor chip 100 flows. Consequently, a sufficient amount of blood is supplied to the vicinity of the sample introduction opening 101. Then, blood is quickly supplied into the sample space 130 from the sample introduction opening 101. A force can be applied to the flow path forming element filled with blood in a compressing direction. Accordingly, blood can be introduced still more from the sample introduction opening into the sample space of the biosensor chip. Consequently, even when the very small sensor chip 100 as described in Patent Document 2 is used, accurate measurement can be achieved. Accordingly, a test of an analyte can be conducted using an extremely small amount of collected blood. The pain of a patient can considerably be alleviated. The lancet needle is pushed by a force acting in the compressing direction to the extent that the tip end of the lancet needle is not in contact with a puncture target surface. Thus, a puncture mark is not blocked up by the tip end of the needle. Consequently, no pain is given to a user again.

In a case where the sensor device 1 is provided with the cap element 16, the contamination of the inner side of the cap element 16 by body fluid can be prevented by adjusting, when a fingertip is detached after the fingertip is punctured, the restoring force of the pressure spring 32 so that the front surface of the flow path forming element 170 is protruded from the leading end of the cap element 16. Thus, the device main element 10 can be utilized repeatedly and hygienically without forcibly replacing the cap element 16.

The case of using the sensor chip 100, on the top surface of which the lancet needle300 is arranged, has been described in the foregoing description of the embodiment. However, a sensor chip 100 can be used, in which the lancet needle 300 is sandwiched between the cover 120 and the substrate 110, each of which is formed of a plate member, and the axis of the lancet needle 300 is located in the sample introduction opening 101 formed between the cover 120 and the substrate 110, as illustrated in, e.g., FIG. 7. Alternatively, the lancet needle 300 can be arranged on the bottom surface (this arrangement is not shown).

Next, a second embodiment of the invention is described below. FIG. 8 is a schematic perspective view of a sensor device 1A. FIG. 9 is a schematic perspective view illustrating a state in which a lid element 210 is opened. FIG. 10 is a schematic cross-sectional view illustrating a state in which the lid element 210 is opened. FIG. 11 is a partially broken schematic perspective view of the sensor element 20 to be mounted in the sensor device 1A shown in FIG. 8. FIG. 12 is a view illustrating a spring connector 270 which connects the drive spring 31 to the pressure spring 32. FIG. 13 is a schematic plan view of the sensor device 1A in which the sensor element 20 is mounted and in which the lid element 210 is opened. FIG. 14 is a schematic cross-sectional view of the sensor device. 1A in which the sensor element 20 is mounted and in which the lid element 210 is opened.

The sensor device 1A according to the second embodiment of the invention includes a device main element 10 in which the sensor element 20 having the sensor chip 100 and a lancet needle 300 is detachably mounted. The sensor element 20 includes the sensor main element 30 formed integrally with the sensor chip 100 and the lancet needle 300, and a sensor body 40 to be attached to the sensor main element 30. The sensor main element 30 is constructed by integrating the lancet needle 300 and the sensor chip 100 with each other by being sandwiched by a fixing member 150 and a support member 160. The sensor main element 30 has a configuration similar to that of the sensor main element 30 according to the first embodiment. The support member 160 does not have the wing portions 161. The entire sensor main element 30 is formed substantially like a cylinder. The length of each of the fixing member 150 and the support member 160 is shorter than that of the sensor chip 100. The terminals 112 of the sensor chip 100 are located at positions protruded rearwardly from the sensor main element 30. The sensor body 40 includes two semicylinder-like body forming members 40a and 40b and does not have the drive spring mounting portion 42, the pressure spring mounting portion 43, and the connector 41. The length of each of the body forming members 40a and 40b is shorter than that of the sensor chip 100 and substantially equal to that of each of the fixing member 150 and the support member 160. Each of the terminals 112 of the sensor chip 100 is located at a position rearwardly protruded from the sensor body 40. A portion at which the flow path forming element 170 is attached to the front surface of the sensor main element 20 is sandwiched by the two body forming members 40a and 40b.

The device main element 10 has the lid element 210 and a casing element 220, in which the measuring circuit and the display mechanism (each of which is not shown) are housed. The lid element 210 is openably and closably attached to the casing element 220 via a mounting member 221. The casing element 220 is shaped like a box the top surface of which is opened. A cylindrical abutting member 230 is provided on the front surface of the casing element 220. The projection opening 231 is provided in the front surface thereof. Bleeding can be facilitated by applying the abutting member 230 onto the puncture target surface to thereby mound a puncture target part in the projection opening 231. However, the abutting member 230 is not indispensable member. The projection opening 231 can be formed directly in the casing element 210.

The casing element 220 has a fixed frame 240 formed into a rectangular-frame-like shape in plan view which is fixed in the casing element 220. In addition, the casing element 220 has a fixed socket 250, and a movable socket 260, which has the sensor element 20 detachably mounted therein and is displaced in the attaching/detaching direction of the sensor element 20, in the fixed frame 240. The movable socket 260 is shaped substantially like a rectangular cylinder. Four inner corners angulate in the rectangular frame in order to temporarily hold the sensor element 20, which is inserted from front, in the cylinder. The fixed socket 250 is formed like a rectangular cylinder and attached to the fixed frame 240 turnably around a turning member 252 at a rearward lower portion thereof. An inner frame of the fixed socket 250 is formed into a shape substantially the same as an outer frame of the movable socket 260. This inner frame of the fixed socket 250 is attached thereto so that the movable socket 260 freely and slidingly moves along the inner frame of the fixed socket 250. A large opening 255 is formed in the top surface of the fixed socket 250.

The top surface of the movable socket 260 is provided with a rail 214, which extends in the attaching/detaching direction of the sensor element 20, in the opening 255 of the fixed socket 250. One end part of a fulcrum support 211 is attached to the mounting member 213 moving on this rail 214 so that the angle formed between the rail 214 and the fulcrum support 211 connected to the lid element 210 changes. The other end part of the fulcrum support 211 is attached to the back surface of the lid element 210 by another mounting member 212 so that the angle formed between the support 211 and the lid element 210 changes. The support 211 is expandable and contractable so that the length thereof is fixed to a certain length when the support 211 is expanded or contracted.

The rail 214 has a stopper (not shown) at each of both ends thereof. When the lid element 210 is closed, the mounting member 213 is displaced frontwardly in a state in which the support 211 is contracted. Thus, the support 211 is located at the front stopper. When the lid element 210 is opened, the support 211 in the contracted state is expanded according to the degree of opening of the lid element 210. When the lid element 211 is opened to the extent that the support 211 is expanded to its maximum, the mounting member 213 is rearwardly displaced on the rail 214 according to the degree of opening of the lid element 210. Subsequently, after the mounting member 213 is displaced to the rear stopper position, the movable socket 260 is pulled upwardly according to the degree of opening of the lid element 210. The fixed socket 250 is turned around the turning member 252. When the lid element 210 is opened, the front part of the movable socket 260 is pulled upwardly (see FIG. 10). When the lid element 210 is closed, the movable socket 260 performs opposite movement and is accommodated in a horizontal position together with the fixed socket 250 (FIGS. 13 and 14). An electric switch (not shown) is arranged at the rear stopper. When the mounting member 213 is displaced to the position of the rear stopper, the switch is turned on. Thus, the measuring circuit is turned on. When the lid element 210 is opened and the mounting member 231 is rearwardly displaced from the position of the front stopper, the switch is turned off. Thus, the measuring circuit is turned off.

The movable socket 260 has a connector 261 which is provided in the cylinder and which is electrically connected to the terminals 112 of the sensor chip 100 in the vicinity of the center in the direction of inserting the sensor element 20. The connector 261 exposes the taking-out terminal 262 to be electrically connected to the measuring circuit. An electrically conductive line (not shown) to be electrically connected to the taking-out terminal 262 and the measuring circuit is drawn out of each of an electrode taking-out opening 263 of the movable socket 260 and the opening 255 of the fixed socket 250. The movable socket 260 has a cylindrical spring arrangement space 265 in which the drive spring 31 and the pressure spring 32 are expanded and contracted in the direction of attaching and detaching the sensor element 20, rearwardly from the electrode connecting connector 261.

The sensor device 1A includes a puncture mechanism configured to cause the sensor element 20 to protrude and hit the puncture target surface such as skin, and a pressing mechanism configured to have the pressure spring 32 that presses the flow path forming element 170, which hits the skin, against the puncture target surface after the skin is punctured, and that assures the flow path 171 until measurement is finished. The puncture mechanism includes the drive spring 31 arranged in rear of the sensor element 20, and the holding mechanism configured to hold the drive spring 31 in a compressed state and to cancel, in response to the drive button 11, a state in which the drive spring 31 is held.

The holding mechanism includes a hook fixing concave portion 45 formed in the bottom surface of the movable socket 260, the hook member 46 interlocking with a drive button 11, the drive button 11, an extrusion pin 12, and a return spring 13. The hook member 46 is formed like a letter "L", and axially supported by a rotating shaft 49 at a part bent like a letter "L". The hook member 46 has a hook 48 caught in the concave portion 45 at one end thereof. Accordingly, when the lid element 210 is opened, the movable socket 260 is displaced rearwardly. Consequently, the hook 48 is caught in the concave portion 45.

The drive button 11 is provided on the rear surface of the casing element 220 and urged in the rearward direction by the return spring 13 provided between the inner side surface of the casing element 220 and the rear surface of the fixed frame 240. The extrusion pin 12 having a leading end for pushing the other end part (i.e., an end part does not have the hook 48) of the hook member 46 is provided on the rear surface of the drive button 11. Accordingly, when the drive button 11 is pushed, the extrusion pin 12 is frontwardly protruded. The hook 48 is disengaged from the concave portion 45. The drive spring 31 is returned by the restoring force to a state in which the drive spring 31 has a natural length. The extrusion pin 12 interlocks with an opening operation of the lid element 210 and is displaced into the shaft of the drive button 11. Then, the leading end of the extrusion pin 12 is located at a position at which the leading end of the extrusion pin 12 does not encumber a turning operation of the fixed socket 250 (see FIG. 10).

In the movable socket 260, the pressure spring 32 is arranged in series with the drive spring 31 via the spring connector 270. In a case where the pressure spring 32 is arranged in the drive spring 31 similarly to the sensor device 1 illustrated in FIG. 1, there are fears that at the assembly of the device, the two springs tangle with each other to thereby obstruct the appropriate assembly of the device, and that in an operation, the two springs tangle with each other to thereby obstruct a proper operation of the device. Such fears can be eliminated by arranging the drive spring 31 and the pressure spring 32 in series with each other.

The drive spring 31 and the pressure spring 32 are arranged in series with each other in the spring arrangement space 265 via the spring connector 270. As illustrated in FIG. 12, the spring connector 270 is formed like a circular-cylinder. The spring connector 270 is displaced in the direction of attaching and detaching the sensor element 20 in the spring arrangement space 265. The spring connector 270 has a projection portion 271 provided on the front surface thereof so that the projection portion 271 holds one end of the pressure spring 32, and that the projection portion271 is inserted into one-end part (i.e., a rear-end part) of the pressure spring 32. The spring connector 270 has a concave portion 272 formed such that one end of the drive spring 32 is inserted into the concave portion 272 and that the concave portion 272 supports the inserted end of the drive spring 32.

The electrode connecting connector 261 has a projection portion 264 provided on the back surface thereof so that the projection portion 264 holds one end of the pressure spring 32 and that the projection portion 264 is inserted into one-end part (i.e., a front-end part) of the pressure spring 32. The pressure spring 32 is supported by the two projection portions 271 and 264 and held in the spring arrangement space 265 of the movable socket 260. The one-end part (front-end part) of the drive spring 31 is fixed to a projection portion 253 provided on the inner surface of the fixed socket 250. The drive spring 31 is supported between the projection portion 253 and the concave portion 272 of the spring connector 270 so as to be held in the spring arrangement space 265.

In a state in which the lid element 210 is opened, the movable socket 260 is rearwardly displaced, so that the leading end of the movable socket 260 is lifted upwardly, as illustrated in FIG. 10. In this state, each of the drive spring 31 and the pressure spring 32 is maintained by the holding mechanism in a compressed state. The sensor element 20 is inserted into and mounted in the movable socket 260 in this state. Then, when the lid element 210 is closed, the fixed socket 260 is set in a horizontal state and brought into a state in which measurement can be performed (see FIGS. 13 and 14).

The drive spring 31 performs the function of causing the sensor element 20 to hit the puncture target surface. Thus, the drive spring 31 has a large restoring force. Consequently, a considerable strong force is required to compress the drive spring 31. However, in the sensor device 1A, the movable socket 260 is rearwardly moved when the lid element 210 is opened. Thus, the drive spring 31 can be compressed by a relatively small force. When the drive spring 31 is returned to a state in which the drive spring 31 has a natural length, the spring connector 270 is swiftly and frontwardly pushed out by a large restoring force. Then, after the sensor element 20 hits the puncture target surface, a force is applied in a direction in which the drive spring 31 is temporarily pressed and contracted by the restoring force of the flow path forming element 170. However, because the drive spring 31 has a large restoring force, the drive spring 31 is not contracted.

The pressure spring 32 is supported by the drive spring 31 via the spring connector 270 after hitting the puncture target surface. Thus, the front surface of the flow path forming element 170 can be pressed against the puncture target surface by the restoring force of the pressure spring 32 itself. At that time, the restoring force of the pressure spring 32 is adjusted to a level so that the tip end of the lancet needle 300 does not touch the puncture target surface. However, because it is sufficient that the flow path forming element 170 is in contact with the puncture target surface, the pressure spring 32 can be returned to a state in which the pressure spring 32 has a natural length. Preferably, the pressure spring 32 has a restoring force whose magnitude is at a level sufficient to the extent that upon completion of measurement, the front surface of the flow path forming element 170 can be located frontwardly from the projection opening 231. That is, it is desired that when the pressure spring 32 is returned to a state in which the pressure spring 32 has a natural length, at least the front surface of the flow path forming element 170 is located frontwardly from the projection opening 231. Thus, blood adhering to the front surface of the flow path forming element 170 is prevented from adhering to the inner surface of the abutting member 230. On the other hand, preferably, the device is maintained in a state in which the leading end surface of the movable socket 260 is protruded from that of the fixed socket 250, as illustrated in FIG. 10, when the lid element 210 is opened. In order to realize this state, the pressure spring 32 requires a restoring force whose magnitude is at a level sufficient to the extent that when the lid element 210 is opened, the movable socket 260 is pushed out without compressing the pressure spring 32 by the own weight of the movable socket 260. That is, even in the case of this sensor device 1A, the spring constant and the length of the pressure spring 32 and the elastic force of the flow path forming element 170 are adjusted comprehensively according to the structure of the device main element 10, e.g., the mass and the length of the movable socket 260, the length of the fixed socket 250, the length of the drive spring 31, and the size of the spring socket 270, and the like.

Next, an operation of the sensor device 1A according to the second embodiment of the invention is described hereinafter with reference to an explanatory view illustrating an operation thereof shown in FIG. 15. The sensor element 20 is mounted in the movable socket 260. When the lid element 210 is closed, the abutting member 230 is pressed against the puncture target surface, e.g., a fingertip M. Then, the drive button 11 is pushed. Thus, the hook 48 is disengaged from the concave portion 45, so that the drive spring 31 and the pressure spring 32 are expanded. Then, the sensor element 20 strongly hits the fingertip. Thus, the tip end of the lancet needle 300 protruded out of the puncture hole 172 of the compressed flow path forming element 170 is inserted into the fingertip M (FIG. 15(a)). Subsequently, the tip end of the lancet needle 300 is removed from the fingertip by the restoring force of the flow path forming element 170. In addition, the device is maintained in a state in which the front surface of the flow path forming element 170 is in contact with the fingertip M by the restoring force of the pressure spring 32 (FIG. 15(b)). Then, upon completion of measurement, when the fingertip is detached from the sensor device 1A, the flow path forming element 170 is frontwardly pushed out by the restoring force of the pressure spring 32. The flow path forming element 170 is stopped (FIG. 15(c)) in a state in which the front surface of the flow path forming element 170 is pushed out of the leading end of the cap element 16. Subsequently, the sensor element 20 is pulled out of the tip-end opening of the puncture portion. Then, measurement is finished.

Thus, the device can be adapted so that after the sensor element 20 hits the fingertip, the flow path forming element 170 is pressed against the puncture target surface by the pressure spring 32 and by arranging the drive spring 31 and the pressure spring 32 in series with each other via the spring connector 270. With this configuration, the assembly of the device can be facilitated. In addition, an operation can reliably be performed.

Apparently, the invention is not limited to the above first and second embodiments. Various embodiments of the invention can be considered. For example, the sensor body 40 constituting the sensor element 20 is not an indispensable constituent element. The sensor main element 30 constituted integrally with the lancet needle 300 can be attached directly to the device main element 10. A puncture mechanism utilizing compressed air or a puncture mechanism using the torque of a motor can be used as a puncture mechanism for causing the sensor element 20 to hit skin.

Next, a third embodiment of the invention is more specifically described hereinafter with reference to the accompanying drawings. FIG. 16 is a schematic perspective view illustrating a sensor device 401 according to the third embodiment of the invention. FIGS. 17 and 18 are schematic cross-sectional views each illustrating a structure of the sensor device 401 to which a lancet-needle-integrated type sensor element420 is attached. FIG. 17 is a schematic cross-sectional view, taken on line A-A' shown in FIG. 16, illustrating a structure of the sensor device according to the third embodiment. FIG. 18 is a schematic cross-sectional view, taken on line B-B' shown in FIG. 16, illustrating the structure of the sensor device according to the third embodiment. FIG. 19 is a partially broken schematic perspective view illustrating the sensor element 420 mounted in the sensor device 401. FIG. 20 is a schematic perspective view illustrating a sensor chip 500 constituting the sensor element 420. FIG. 21 is a partially enlarged schematic explanatory view of the sensor element 420. FIG. 22 is an explanatory view illustrating an operation of the sensor device 401.

The sensor device 401 according to the third embodiment of the invention includes a device main element 410 in which the sensor element 420 having the sensor chip 500 and a lancet needle 600 is detachably mounted. The sensor element 420 includes the sensor main element 430 formed integrally with the sensor chip 500 and the lancet needle 600, and a sensor body 440 to be attached to the device main element 410. The sensor main element 430 is constructed by integrating the lancet needle 600, which includes a puncture needle for hurting skin to cause bleeding or a miniature cutting tool, and the sensor chip 500 with each other by a fixing member 550 and a support member 560. Further, the sensor element 420 is provided as what is called a disposable product.

As illustrated in FIG. 20, the sensor chip 500 has a structure in which a cover 520 and a substrate 510, each of which is formed of an insulating plate member, are stuck to each other with an adhesive agent layer 540 so as to form a sample space 530 between the cover 4120 and the substrate 510. A sample introduction opening 501 connected to the sample space 530 is opened in an end part of the sensor chip 500. An air hole 541 connected to the sample space 530 and the outside of the sensor chip 500 is provided at a rear end part of the sample space 530 so as to extend to both sides of the rear end part of the sample space 530.

A pair of electrodes 511 is formed on the substrate 510 so as to face the sample space 530. Voltage taking-out terminals 512 electrically connected to the electrodes 511 with electrical conductors, respectively, are formed at an end part of the substrate , which is opposite to the sample introduction opening 501. The cover 520 is provided with a reagent layer 521 which reacts with a sample (more specifically, body fluid such as blood) and is formed to face the sample space 530. A biosensor includes the pair of electrodes 511 and the reagent layer 521. Electrodes 513 for a liquid junction detection circuit that detects a sample introduced into the sample space 530 are formed on the substrate to face the rear end of the sample space 530. The electrodes 513 are electrically conducted to voltage taking-out terminals 514 formed in the vicinity of the voltage taking-out terminals 512, respectively. A chip including the substrate and the cover manufactured by bending plate members as described by the Patent Document 2 is exemplified as the sensor chip 500. However, according to the invention, a structure other than that described by the Patent Document 2 can be employed as the structure of the biosensor. As long as a structure is such that the sample introduction opening 501 is provided between the substrate 510 and the cover 520 and that a sample is introduced from the sample introduction opening 501, the invention can be applied to such a structure.

The lancet needle 600 is arranged on the cover 520 of the sensor chip 500 and is sandwiched between a fixing member 550 and a support member 560 and fixed to the sensor chip 500. The lancet needle 600 is fixed thereto by protruding a tip end of the lancet needle 600 from a tip end of the sensor chip 500 in order to hurt skin. The lancet needle 600 is arranged such that an axis of the lancet needle 600 is substantially parallel to a direction in which the sample space 530 is provided so as to quickly guide to the sample introduction opening 501 body fluid that comes out from the skin, and that the axis thereof is located close to, preferably, just above the sample introduction opening 501.

The support member 560 has a function of supporting the sensor chip 500 from the bottom surface thereof, and is made of, e.g., a plastic material. The fixing member 550 has a function of protecting the sensor chip 500 from the direction of the top surface thereof, and is manufactured by being made of, e.g., a plastic material. The fixing member 550 has also the function of fixing the lancet needle 600. A cutout (not shown) for fixing the lancet needle 600 is formed in the bottom surface. Apparently, the fixing member 550 and the support member 560 can be formed integrally with each other.

The sensor main element 430 is attached to the sensor body 440 formed like a substantially cylinder, as illustrated in, e.g., FIG. 19. The sensor body 440 is used to attach the sensor chip to the device main element 410. In the sensor body 440, a connector 441 for electrically connecting the terminals 512 of the sensor chip 500 to a measuring circuit (not shown) of the device main element 410 and for electrically connecting the terminals 514 to a liquid junction detection circuit (not shown) is provided. A cylindrical drive spring mounting portion 442, whose outside diameter is smaller than that of the leading-end-side part of the sensor body 440, is provided at the rear-end-side part of the sensor body 440. A cylinder inside portion of the drive spring portion 442 is a tapered sensor element holding portion 443 whose diameter is gradually reduced towards the end of the sensor body 440. The tapered sensor element holding portion 443 constitutes a part of the holding mechanism for attaching the sensor element 420.

The device main element 410 includes a puncture mechanism portion 450 for forming an enclosed space by pressing the puncture target surface thereagainst and for causing the sensor element 420 attached thereto to puncture skin in the enclosed space, a decompressing mechanism portion 460 for decompressing the enclosed space, a conversion circuit for converting an electromotive force generated in the sensor chip 500 to a measured value, a display mechanism (not shown) for displaying the measured value, a liquid junction detection circuit (not shown) for detecting that a sample is introduced into the sample space 530, and the like.

The puncture mechanism portion 450 includes a puncture mechanism chamber 451 whose end part is manufactured like a cylinder, and a puncture mechanism for causing the sensor element 420 to hit the puncture target surface. The puncture mechanism chamber 451 forms an enclosed space by pressing the puncture target surface, such as a fingertip, against a leading-end opening 416 thereof. The puncture mechanism includes, e.g., a spring mechanism. The spring mechanism includes a drive spring 431 provided at a rear portion of the sensor element 420, and a holding mechanism for holding the drive spring 431 in a compressed state and for canceling, in response to a drive button 411, a held state in which the drive spring is held. The drive spring 431 has one-end part fixed to a rear wall surface 452 of the puncture mechanism chamber 451. The puncture mechanism portion is configured such that the drive spring mounting portion 442 of the sensor element 420 can be inserted into the chamber from the other-end-side part of the drive spring 431.

As long as the holding mechanism is a mechanism that holds the drive spring 431 in a compressed state in the enclosed space, that cancels the held state in response to an operation of the button or the like and that causes the sensor element 420 to hit the puncture target surface, the holding mechanism can have any configuration. The illustrated holding mechanism includes the concave portion 445 provided in the continuous connection portion 444 for fixing the hook, the hook member 446 interlocked with the drive button 411, the holding spring 447, and the holding member 449 and the sensor element holding portion 443 provided on the continuous connection portion 444 so as to frontwardly protrude therefrom. The drive button 411 is upwardly urged by the holding spring 447. The hook 448 provided at the leading end of the hook member 446 is caught in the concave portion 445. The holding member 449 is manufactured like a circular truncated cone whose diameter is gradually reduced towards an end thereof. The drive spring 431 is maintained in a compressed state by inserting the holding member 449 into the sensor element holding portion 443 (i.e., in the cylinder of the drive spring mounting portion 442). The continuous connection member 444 is frontwardly pushed out to thereby frontwardly push out the holding member 449 (or the continuous connection member 444 on which the rear end surface of the drive spring mounting portion 442 abuts) as well as the sensor element 420. Thus, the drive spring 431 is released.

In the case of the sensor device 401, in order to maintain the enclosed space in a decompressed condition even in a punctured state, i.e., in a state in which the sensor element 420 hits the puncture target surface, the drive spring mounting portion 442 is protruded slightly rearwardly from the opening 453 opened in the rear wall surface 452 of the puncture mechanism chamber 451 in the punctured state. An O-ring made of fluorine resin (not shown) is provided in the opening 453. Thus, the airtightness of the puncture mechanism chamber 451 and the slidability of the drive spring mounting portion 442 are assured.

The compressing mechanism portion 460 performs the functions of bringing the puncture mechanism chamber 451 into a compressed state and introducing, after punctuation, discharged body fluid from the sample introduction opening 501 to the sample space 530. That is, according to the invention, the entire biosensor chip 500 is put in reduced-pressure environment to thereby evacuate the air contained in the sample space 530 from the air hole 541, and then body fluid is quickly introduced thereinto. As long as the decompressing mechanism portion performs such a function, the practical configuration of the decompressing mechanism portion is insignificant. For example, an air damper can be used as the decompressing mechanism portion. The decompressing mechanism of the illustrated decompressing mechanism portion 460 includes a tubular cylinder chamber 461 capable of achieving an airtight state, a piston 462 housed in the cylinder chamber 461, a piston spring 463 for driving the piston 462, and a piston holding mechanism for maintaining the piston spring 463 in a compressed state, and canceling the maintained state by the drive button 411. The cylinder chamber 461 communicates with the puncture mechanism chamber 451 via, e.g., a communication hole 464 provided in a base end of the cylinder chamber 461. Thus, the airtight state of the cylinder chamber 461 integrated with the puncture mechanism chamber 451 is assured. An electromagnetic valve 465 for releasing the inside of the cylinder chamber 461 to atmospheric pressure is provided at the base end of the cylinder chamber 461. The electromagnetic valve 465 is controlled by the liquid junction detection circuit. When it is detected that a sample is introduced into the sample space 530, the electromagnetic valve 465 is released. That is, in a state in which the electromagnetic valve 465 is closed, the airtight state of the puncture mechanism chamber 451 and the cylinder chamber 461 is assured by blocking up the leading-end opening 416 of the puncture mechanism chamber 451 with the puncture target surface such as a fingertip. When the electromagnetic valve 465 is released, air is introduced therefrom, so that the puncture mechanism chamber 451 and the cylinder chamber 461 are released to atmospheric pressure. In addition, an O-ring made (not shown) of fluorine resin or the like is provided in the opening of the rear wall surface 467 of the cylinder chamber 461 through which the piston rod 466 is inserted. Thus, the airtightness of the cylinder chamber 461 and the slidability of the piston rod 466 are assured.

The piston 462 is housed in the cylinder chamber 461 and urged by the restoring force of the piston spring 463 so as to expand the space of the cylinder chamber 461. The piston holding mechanism includes the piston rod 466 provided at the rear end side of the piston 462, the hook fixing concave portion 445 provided in the continuous connection member 444 to which one end of the piston rod 466 is fixed, and the hook member 446 and the holding spring 447 interlocked with the drive button 411. That is, the device is configured so that the holding mechanism for the piston spring 463 and the holding mechanism for the drive spring 431 operate simultaneously. Each of the compressed piston spring 463 and the compressed drive spring 431 is restored to a natural state by pushing down the drive button 411. Consequently, the leading end of the holding member 449 pushes the sensor element holding portion 443, so that the drive spring 431 is restored. Thus, the sensor element 420 is swiftly pushed out towards the puncture target surface. Simultaneously with this, in the airtight state, the space of the cylinder chamber 461 is expanded. Consequently, a negative pressure is generated in the cylinder chamber 461. This results in the decompression of the puncture mechanism chamber 451 (and the cylinder chamber 461).

At that time, it is desirable that the puncture mechanism chamber 451 is slowly decompressed by, e.g., setting the restoring force of the piston spring 463 to be weaker than that of the drive spring 431. That is, it is necessary to cause the lancet needle 600 to hit the puncture target surface by making the sensor element 420 protrude rapidly. On the other hand, it is desired that the decompressing mechanism portion 460 slowly drives the piston 462 like an air damper, and that a negative pressure is gradually generated. Differently from this, in a case where the piston 462 is driven by a restoring force similar to that of the drive spring 431, a negative pressure is abruptly generated. Consequently, a large amount of body fluid flows into the sample space 530 at a time. This results in increase of the risk of entry of air bubbles into the sample space 530 so as to cause a measurement failure, and the risk of enhancement of burden to a test subject, i.e., pain at bleeding.

The continuous connection member 444 to which the piston rod 466 and the holding member 449 are fixed is interlocked with each of lock levers 414 respectively protruding from each of the left and right side surfaces of the device main element 410. That is, the piston spring 463 is compressed by operating the lock levers 414. Thus, the hook 448 is caught n the concave portion 445 of the continuous connection member 444. In addition, the holding member 449 is backwardly retreated. The sensor element 420 is mounted in the puncture mechanism portion 450. Thus, the sensor element 420 is set in the device main element 410 while the drive spring 431 is put in a compressed state.

Next, an operation of the sensor device 401 according to the third embodiment of the invention is described hereinafter with reference to an explanatory view shown in FIG. 22 illustrating an operation thereof. First, each lock lever 414 is backwardly pulled in the sensor device 401 brought in a natural state which the piston spring and the drive spring are restored as illustrated in FIG. 22(a). Thus, the piston spring 463 is compressed, so that the hook 448 of the holding mechanism is caught in the concave portion 445 of the continuous connection member 444. Then, the sensor element 420 is inserted from the leading-end opening 416 of the puncture mechanism chamber 451. Thus, while the holding member 449 is inserted into the sensor element holding portion 443 and the drive spring. 431 is compressed, the sensor element 420 is set in the device main element 410 (see FIG. 22(b)). When the sensor element 420 is set in the device main element 410, the measuring circuit is turned on. In this state, the electromagnetic valve 465 is opened, so that the puncture mechanism chamber 451 and the cylinder chamber 461 are at atmospheric pressure. Then, as illustrated in FIG. 22 (c), the drive button 411 is pushed down in a state in which the leading-end opening 416 of the puncture mechanism chamber 451 is blocked up by pressing the fingertip M against this opening 416. Then, the electromagnetic valve 465 is closed to form an enclosed space. In addition, the state in which the hook is caught is canceled. Then, the continuous connection member 444 is drawn to the front side by the restoring force of the piston spring 463. Thus, the holding member 449 (or the continuous connection member 444) pushes out the sensor element 420. When the sensor element 420 is pushed out, the drive spring 431 is restored. The sensor element 420 is swiftly and frontwardly protruded due to the restoring force of the drive spring. Thus, the lancet needle 600 is inserted into the fingertip M. Because the electromagnetic valve 465 is closed due to the depression of the drive button 411, the puncture mechanism chamber 451 is gradually decompressed while the piston spring 463 is restored. Then, blood flows out of the punctured part. The blood is transmitted on the lancet needle 600 and guided from the sample introduction opening 501 to the sample space 530 from which air is evacuated. When the sample space 530 is filled with blood, the liquid junction detection circuit is turned on, so that the electromagnetic valve 465 is released. Subsequently, the fingertip M is detached from the opening 416. The sensor element 420 is taken out of the device main element 410. Thus, the measurement is finished. When the sensor element 420 is removed from the device main element 410, the measuring circuit is turned off. The removed sensor element 420 is discarded.

Thus, in the sensor device 401 according to the third embodiment of the invention, body fluid drawn out of the punctured part is quickly guided into the sample space 530 provided in the sensor chip 500 by putting the sensor chip 500, whose needle is inserted into the puncture target surface, in the reduced pressure environment. Consequently, even when the very small sensor chip 500 as described in Patent Document 2 is used, accurate measurement can be achieved. Accordingly, a test of an analyte can be conducted using an extremely small amount of collected blood. The pain of a patient can be alleviated. More particularly, the electromagnetic valve 465 is controlled by the liquid junction circuit in the device 401. The device has a decompression release mechanism for releasing the puncture mechanism chamber 451 to atmospheric pressure when a necessary amount of blood is collected. Thus, body fluid (or blood), whose amount is equal to or more than a necessary amount, is prevented from being collected. In addition, it can be sensed by a fingertip or the like that the collection of sample is completed before a measured value is displayed. Thus, the device according to the invention can eliminate a user's concern about whether the collection of blood is favorably performed.

In the foregoing description of the third embodiment, the case of using the sensor chip 500, on the top surface of which the lancet needle 600 is arranged, has been described. However, for example as shown in FIG. 23, the device can employ the sensor chip 500 in which the lancet needle 600 is sandwiched between the cover 520 and the substrate 510 and in which the axis of the lancet needle 600 is located in the sample introduction opening 501 formed between the cover 520 and the substrate 510. Alternatively, the lancet needle 600 can be arranged on the bottom surface.

Apparently, the invention is not limited to the above third embodiment. Various embodiments can be considered. For example, the sensor body 440 constituting the sensor element 420 is not an indispensable constituent element. The sensor main element 430 constituted integrally with the lancet needle 600 can be attached directly to the device main element 410. A puncture mechanism utilizing compressed air or a puncture mechanism using the torque of a motor can be used as a puncture mechanism for causing the sensor element 420 to hit skin. Such a puncture mechanism and a decompressing mechanism such as an air damper can be driven simultaneously with the pressing of a depression button.

In addition, as described in the foregoing description, the sensor device can be formed as an integrated type device by incorporating the decompressing mechanism into the main element. However, according to the invention, it is not always necessary to incorporate a decompressing mechanism portion into the main element. A decompressing mechanism portion can be provided outside the main element. The device can be constructed so as to depressurize the puncture mechanism chamber using, e.g., an exhaust tube or the like.

Incidentally, it should be understood that the expressions "sensor chip" and "biosensor chip" are synonymous ones in the description of each of the above embodiments.

Although the invention has been described in detail with reference to specific embodiments, it will be apparent to those skilled in this art that various change and modifications can be made therein without departing from the spirit or scope of the invention. The invention is based on Japanese Patent Application (No. 2007-046323) filed February 26, 2007, Japanese Patent Application (No. 2007-046327) filed February 26, 2007, and Japanese Patent Application (No. 2007-200248) filed July 31, 2007, and their contents are incorporated herein as reference.

### Industrial Applicability

According to the invention, a measuring device can be provided, which can reduce a physical burden to a patient. This measuring device can reliably perform measurement even when an amount of collected blood is small. Thus, a very small biosensor chip can be used. In addition, a sensor device itself is miniaturized. Consequently, a sensor device can be provided, which is advantageous in portability.

## Claims

1. A sensor device for detachably mounting a needle-integrated type sensor element, the sensor element having a sensor chip, a lancet needle fixed to an end portion of the sensor chip, and a flow path forming element provided compressibly and restorably at the end portion of the sensor chip, to which the lancet needle is fixed,
the sensor device comprising:
a puncture mechanism for causing the sensor element to puncture a target; and
a pressing mechanism, which is different from the puncture mechanism, for urging the sensor element to an extent that the sensor element is prevented from being detached from the target by a restoring force of the flow path forming element after the target is punctured.

2. The sensor device according to claim 1, further comprising:
a cap element provided with an opening for causing the lancet needle to puncture the target, wherein
when the target is detached from the opening of the cap element after the target is punctured, the pressing mechanism urges the sensor element to an extent that at least a front surface of the flow path forming element is protruded from the opening of the cap element.

3. The sensor device according to claim 1 or 2, wherein
the pressing mechanism and/or the puncture mechanism includes a spring mechanism.

4. The sensor device according to any one of claims 1 to 3, wherein
the flow path forming element is made of a polymer material having airtightness and elasticity.

5. The sensor device according to any one of claims 1 to 4, wherein
the sensor chip comprises a cover and a substrate, each of which is formed of a plate member, and
a sample space in which a sample and a reagent layer react with each other is provided between the cover and the substrate.

6. A sensor device having a needle-integrated type sensor element detachably mounted therein, the sensor element having a biosensor chip, and a lancet needle fixed to one end portion of the biosensor chip,
the sensor device comprising:
a puncture mechanism for forming an airtight space by applying a puncture target surface thereto and for causing the sensor element to hit the puncture target surface in the airtight space; and
a decompressing mechanism for decompressing the enclosed airtight space, wherein
the airtight space is decompressed by driving the decompressing mechanism; and
a sample is guided into a sample space from a sample introduction opening of the biosensor chip.

7. The sensor device according to claim 6, further comprising:
a detection mechanism for detecting that a sample is guided into the sample space in a state in which the airtight space is maintained; and
a decompression release mechanism for releasing the inside of the puncture mechanism chamber to atmospheric pressure with interlocking with the detection mechanism.

8. The sensor device according to claim 6 or 7, wherein
the decompressing mechanism comprises:
a piston mechanism;
a spring mechanism for driving the piston mechanism; and wherein
a cylinder chamber constitutes the piston mechanism.

9. The sensor device according to any one of claims 6 to 8, wherein
the puncture mechanism comprises a spring mechanism.

10. The sensor device according to any one of claims 6 to 9, wherein
the biosensor chip comprises a cover and a substrate, each of which is formed of a plate member, and
a sample space in which a sample and a reagent layer react with each other is provided between the cover and the substrate.
